(19) Europäisches Patentamt
European Patent Office
Office européen des brevets

(11) **EP 2 685 241 A1**

(12) **EUROPEAN PATENT APPLICATION**
published in accordance with Art. 153(4) EPC

(43) Date of publication:
**15.01.2014 Bulletin 2014/03**

(51) Int Cl.:
***G01N 21/78*** *(2006.01)*

(21) Application number: **12757765.8**

(22) Date of filing: **29.02.2012**

(86) International application number:
**PCT/JP2012/001388**

(87) International publication number:
**WO 2012/124269 (20.09.2012 Gazette 2012/38)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **11.03.2011 JP 2011054711
11.03.2011 JP 2011054713
18.03.2011 JP 2011061279**

(71) Applicant: **Panasonic Healthcare Co., Ltd.
Ehime 791-0395 (JP)**

(72) Inventors:
• **MIKI, Hideshi
Osaka 540-6207 (JP)**

• **TANAKA, Motohiro
Osaka 540-6207 (JP)**
• **MIKI, Hideshi
Osaka 540-6207 (JP)**
• **KOIKE, Yoshitomi
Osaka 540-6207 (JP)**

(74) Representative: **Eisenführ Speiser
Patentanwälte Rechtsanwälte PartGmbB
Johannes-Brahms-Platz 1
20355 Hamburg (DE)**

(54) **NITROGEN OXIDE CONCENTRATION MEASUREMENT DEVICE**

(57) This nitrogen oxide concentration measurement device measures a nitrogen oxide concentration by shining light on a sensor produced by distributing a metal complex of porphyrin or a derivative thereof over a base material, and detecting the amount of light of a specific wavelength, said device comprising a light source that shines light at a nitrogen oxide concentration measurement sensor, a light detector that detects the amount of light of a specific wavelength out of all the light transmitted or reflected by the nitrogen oxide concentration measurement sensor, and a controller that corrects the detection result at the light detector so as to match the amount of light of a specific wavelength outputted from the light detector during exposure to a zero gas to a corrected value.

FIG. 1

EP 2 685 241 A1

**Description**

TECHNICAL FIELD

The present invention relates to a nitrogen oxide concentration measurement device for measuring the concentration of a gas such as nitric oxide, which serves as an index of the extent of inflammation of bronchial tubes caused by asthma or the like.

BACKGROUND ART

Ever since nitric oxide (hereinafter referred to as "NO") was discovered as the main ingredient of a muscle relaxing factor, and its physiological action was revealed, the result of measuring NO concentration has been utilized a neural information transmitter or an infection marker.

In particular, it has been noted that the result of measuring NO concentrations in exhaled air can be used as an index of the extent of respiratory tract infection due to allergies and asthma, which has been on the rise in recent years, making possible a non-invasive diagnosis of disease that is less of a burden to the patient. The NO gas concentration in exhaled air is from 2 to 20 ppb in healthy people, but it is known that it can roughly triple in patients with airway inflammation due to asthma or allergies. Accordingly, if exhaled NO gas is measured, the result can be used as a guideline for treating asthma, such as in determining the extent of airway inflammation in the patient, or deciding on dosages of asthma drugs.

With a conventional method for measuring the concentration of NO in exhaled air, a chemiluminescence NO measurment device was used in which the NO contained in the patient's exhaled air was reacted with ozone under reduced pressure, and the light emitted at that point was detected. With this a chemiluminescence method, however, a problem was that maintenance was difficult because of the need for expensive peripheral devices such as an ozone generator.

Accordingly, for an asthma patient to be able to self-manage his asthma by measuring the NO concentration every day at home or in a hospital, a device is necessary that is inexpensive and compact, has excellent gas selectivity, and measures the NO concentration in exhaled air at high sensitivity.

For example, Patent Literature 1 discusses technology whereby a detecting film composed of a polymer in which porphyrin has been dispersed is formed on an alumina base material, light reflected by the detecting film is detected as detection light, and the concentration of the measured gas is calculated on the basis of the change in the detection light before and after the detecting film comes into contact with the measured gas.

In Patent Literature 2, the product of supporting porphyrin (using cobalt as the central metal) on a base material is used as an NO concentration measurement sensor in which porphyrin is used as the detecting film.

Patent Literature 3 discloses a sensor module comprising an automobile exhaust gas temperature sensor and a controller that corrects variance in sensitivity between exhaust gas temperature sensor lots.

CITATION LIST

PATENT LITERATURE

Patent Literature 1: Japanese Laid-Open Patent Application H10-62350
Patent Literature 2: WO2010/061536
Patent Literature 3: Japanese Laid-Open Patent Application H9-264768

SUMMARY

TECHNICAL PROBLEM

However, the following problems are encountered with the above-mentioned conventional nitrogen oxide concentration measurement devices.

Specifically, with the nitrogen oxide concentration measurement devices disclosed in the above publications, a material (alumina) that reflects light in order to detect reflected light, or a material (such as paper (cellulose) or nonwoven cloth in which the fibers are PET (polyethylene terephthalate)) that transmits light in order to detect transmitted light, is used as the base material of a sensor for measuring NO concentration, but particularly when a nonuniform base material with intertwined fibers is used, such as paper or nonwoven cloth, there is the risk that the sensitivity will vary from one sensor to the next depending on the thickness of the base material and other factors, even though the porphyrin solution is dripped under the same conditions. As a result, there may be a difference in the amount of light absorbed between sensors of high and low porphyrin concentrations, which creates variance in sensor sensitivity, and there is the risk that the wrong concentration value will be outputted as the measurement result even though NO is contained in the same concentration in a patient's exhaled air.

It is an object of the present invention to provide a nitrogen oxide concentration measurement device with which there is less difference in detection caused by variance in  sensitivity from one sensor to the next, making it possible to measure the concentration of NO or other nitrogen oxides very precisely.

The nitrogen oxide concentration measurement device measures a nitrogen oxide concentration by shining light on a sensor produced by distributing a metal complex of porphyrin or a derivative thereof over a base material, and detecting the amount of light of a specific wavelength, said device comprising a light source that shines light at a nitrogen oxide concentration measurement sensor, a light detector that detects the amount of light of a specific wavelength out of all the light transmitted or reflected by the nitrogen oxide concentration measurement sensor, and a controller that corrects the detection result at the light detector so as to match the amount of light of a specific wavelength outputted from the light detector during exposure to a zero gas to a corrected value.

BRIEF DESCRIPTION OF DRAWINGS

FIG. 1 is a block diagram of the configuration of the nitrogen oxide concentration measurement device pertaining to an embodiment of the present invention;

FIG. 2 shows the structure of a porphyrin (CoTPP) supported on an NO concentration measurement sensor used in the nitrogen oxide concentration measurement device in FIG. 1;

FIG. 3 is a graph of the absorption spectrum of the NO concentration measurement sensor used in the nitrogen oxide concentration measurement device in FIG. 1;

FIG. 4 is a simplified diagram of a method for manufacturing the NO concentration measurement sensor used in the nitrogen oxide concentration measurement device in FIG. 1;

FIG. 5 is a graph of correlation lines expressing the correlation between the NO detection sensitivity of an NO concentration measurement sensor and the amount of 415 nm light detected during zero gas correction in a 415 nm light quantity detector of the nitrogen oxide concentration measurement device in FIG. 1, under conditions of NO concentration of 10, 100, and 500 ppb;

FIG. 6 is a graph of the relation between the slope of the correlation lines in FIG. 5 and the output of a differential processor;

FIG. 7 is a graph of a method for correcting measurement values in the nitrogen oxide concentration measurement device in FIG. 1, under conditions of an NO concentration of 100 ppb;

FIG. 8 is a graph of the relation between the height of a drip nozzle and NO concentration under a 100 ppb NO concentration environment;

FIG. 9 is a graph of the correlation between the amount of 415 nm light during zero gas correction and NO sensitivity under a 100 ppb NO concentration environment;

FIG. 10 is a block diagram of the configuration of the nitrogen oxide concentration measurement device pertaining to another embodiment of the present invention;

FIG. 11 is a block diagram of the detailed configuration of a nitrogen oxide concentration measurement device;

FIG. 12 is a flowchart of NO concentration measurement in a nitrogen oxide concentration measurement device;

FIG. 13 is a calibration curve showing the relation between NO concentration and a concentration signal;

FIG. 14 is a graph of concentration signals obtained by using various NO concentration measuring sensors for gas containing a known NO concentration and an initial signal obtained with three different NO concentration measurement sensors;

FIG. 15 is a block diagram of the configuration of the nitrogen oxide concentration measurement device pertaining to an embodiment of the present invention;

FIG. 16 is a graph of variance in NO sensitivity prior to feedback control of a NO concentration measurement sensor for the amount of 415 nm light detected by the 415 nm light quantity detector of the nitrogen oxide concentration measurement device in FIG. 15, under conditions of NO concentration of 20, 100, and 200 ppb;

FIG. 17 is a graph of the change in NO detection sensitivity when the LED current value of the LED light source in FIG. 15 is varied, under conditions of NO concentration of 100 ppb;

FIG. 18 is a graph of variance in NO sensitivity after feedback control when the measurement results for the various NO concentration measurement sensors shown in FIG. 16 are compiled into a constant value for 415 nm light amount (1.0 V);

FIG. 19 is a graph of an example of a calibration curve for calculating the NO concentration (ppb) from NO sensitivity (V);

FIG. 20 is a block diagram of the configuration of the nitrogen oxide concentration measurement device pertaining to Working Example 1 of the present invention;

FIG. 21 is a block diagram of the configuration of the nitrogen oxide concentration measurement device pertaining to Working Example 2 of the present invention;

FIG. 22 is a block diagram of the configuration of the nitrogen oxide concentration measurement device pertaining to Working Examples 3 and 4 of the present invention;

FIG. 23 is a graph of sensor sensitivity when exposed to $NO_2$ with a concentration of 250 ppb;

FIG. 24 is a graph of the result of performing slope correction on the measurement results of 100 ppb NO sensitivity to the 415 nm light amount; and

FIGS. 25a to 25c are graphs of the results of performing slope correction in three zones obtained by diving the graph in FIG. 24 into three zones A, B, and C.

DESCRIPTION OF EMBODIMENTS

In the following embodiments, nitrogen oxide concentration measurement devices 10, 110, and 210 will be described as an example of the nitrogen oxide concentration measurement device of the present invention.

Embodiment 1

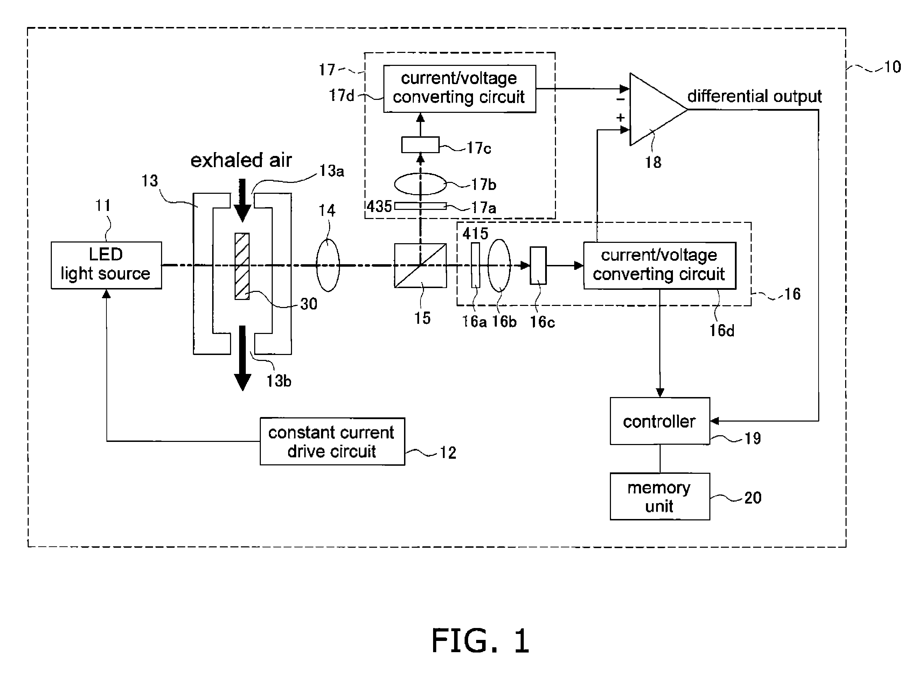

A nitrogen oxide concentration measurement device 10 pertaining to an embodiment of the present invention will be described through reference to FIGS. 1 to 9.

In this embodiment, the output value from a current/voltage converting circuit 16d shown in FIG. 1 means the voltage value (V) corresponding to the amount of light with a 415 nm wavelength, while the output value from a differential processor 18 means the voltage value (V) corresponding to sensitivity.

Configuration of Nitrogen Oxide Concentration Measuring Device 10

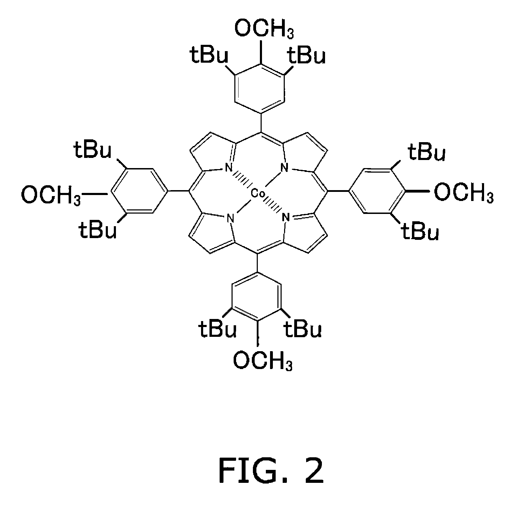

As shown in FIG. 1, the nitrogen oxide concentration measurement device 10 pertaining to this embodiment is a device that measures the concentration of NO contained in the exhaled air of an asthma patient, etc., by introducing exhaled air into a chamber 13 in which a nitric oxide (NO) concentration measurement sensor 30 is installed, and detecting the change in the amount of light with the peak wavelength of the absorption spectrum, which varies with the degree of reaction between NO and a cobalt metal complex of porphyrin (CoT(dtBu)PP) (see FIG. 2) supported on the NO concentration measurement sensor 30. As shown in FIG. 1, the nitrogen oxide concentration measurement device 10 comprises a zero gas corrector 99, an LED light source 11, a constant current drive circuit 12, the chamber 13, a lens 14, a dichroic mirror 15, a 415 nm light quantity detector (light detector, first detector) 16, a 435 nm light quantity detector (light detector, second detector) 17, the differential processor 18, a sensitivity correction processor (controller) 19, and a memory unit 20.

The zero gas corrector 99 calibrates the output of the differential processor 18 with respect to 0 ppb by sending gas of 0 ppb to the NO concentration measurement sensor 30 provided inside the chamber 13.

The LED light source 11 irradiates the NO concentration measurement sensor 30 provided inside the chamber 13 with light under specific conditions.

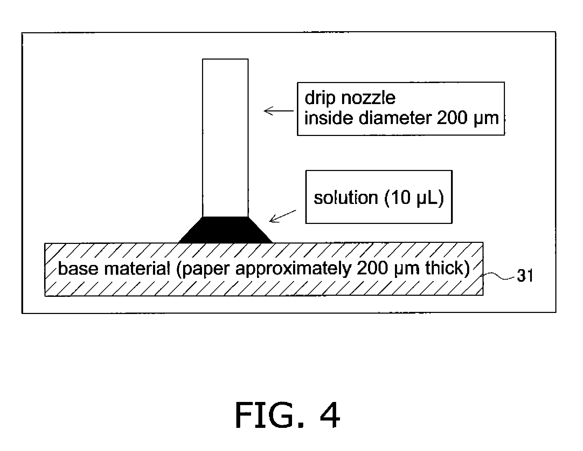

The constant current drive circuit 12 is a circuit for determining the current applied to the LED light source 11, and adjusts the amount of light emitted from the LED light source 11 by varying the current value to be applied. In this embodiment, the constant current drive circuit 12 applies initialization current of 700 mA to the LED light source 11 during initialization of the NO concentration measurement sensor 30, and applies a measurement current of 40 mA in the measurement of NO concentration. In this embodiment, initialization refers to a process in which a porphyrin solution is dripped onto a base material 31 (see FIG. 4), after which cobalt, which is the central metal of the porphyrin, is reduced from trivalent to divalent. The NO concentration measurement sensor 30 on which porphyrin is supported will be described in detail at a later stage.

The chamber 13 is a housing inside of which the NO concentration measuring sensor 30 is installed, and has a gas inlet 13a into which exhaled air or the like is introduced, and a gas outlet 13b for discharging the same.

The light that has been emitted from the LED light source 11 and transmitted from the NO concentration measurement sensor 30 installed in the chamber 13 is guided by the lens 14 to the dichroic mirror 15, which is disposed at a later stage.

The dichroic mirror 15 reflects light of a specific wavelength (at least 425 nm in this embodiment) out of the light transmitted by the NO concentration measurement sensor 30 and received via the lens 14, and transmits light of other wavelengths. This separates just the light of a specific wavelength (at least 435 nm) from the transmitted light.

The 415 nm light quantity detector 16 takes out light with a wavelength near 415 nm from the light transmitted by the dichroic mirror 15, and detects the amount of this light (hereinafter referred to as the 415 nm light quantity). As shown in FIG. 1, the 415 nm light quantity detector 16 has a bandpass filter (BPF) 16a, a lens 16b, a photodiode (PD) 16c, and the current/voltage converting circuit 16d.

The bandpass filter 16a transmits just light with a wavelength near 415 nm out of the light transmitted by the dichroic mirror 15.

The lens 16b guides the light near 415 nm transmitted by the bandpass filter 16a to the photodiode 16c disposed at a later stage.

The photodiode 16c converts the light near 415 nm coming from the lens 16b into a current signal and outputs this signal.

The current/voltage converting circuit 16d converts the electrical signal coming from the photodiode 16c from a current value into a voltage value, and outputs the result to differential processor 18 and the sensitivity correction processor 19.

The 435 nm light quantity detector 17 takes out the light with a wavelength near 435 nm from the light reflected at the dichroic mirror 15, and detects the amount of this light (hereinafter referred to as the 435 nm light quantity). As shown in FIG. 1, the 435 nm light quantity detector 17 has a bandpass filter (BPF) 17a, a lens 17b, a photodiode (PD) 17c, and a current/voltage converting circuit 17d.

The bandpass filter 17a transmits just light with a wavelength near 415 nm out of the light reflected at the dichroic mirror 15.

The lens 17b guides the light near 415 nm transmitted by the bandpass filter 17a to the photodiode 17c disposed at a later stage.

The photodiode 17c converts the light near 415 nm coming from the lens 17b into a current signal and outputs this signal.

The current/voltage converting circuit 17d converts the electrical signal coming from the photodiode 17c from a current value into a voltage value, and outputs the result to the differential processor 18 and the sensitivity correction processor 19.

The differential processor 18 outputs the difference between the 415 nm light quantity and the 435 nm light quantity (detection sensitivity) that were converted into voltage values  at the current/voltage converting circuit 16d on the 415 nm light quantity detector 16 side and the current/voltage converting circuit 17d on the 435 nm light quantity detector 17 side.

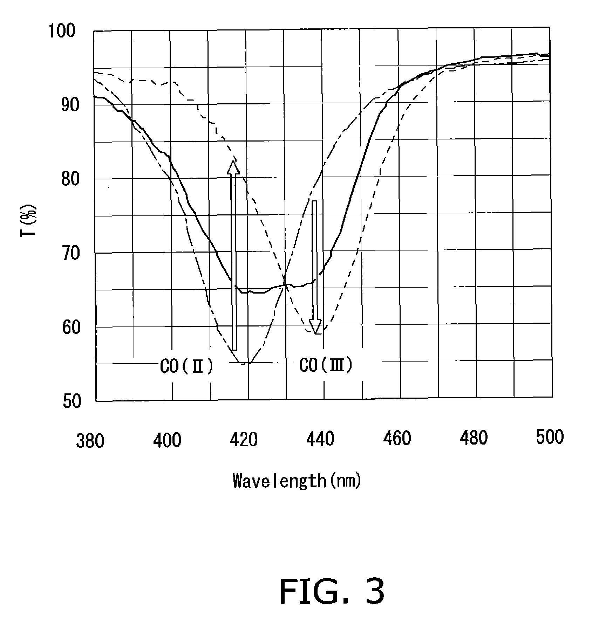

Here, as shown in FIG. 3, the porphyrin supported on the NO concentration measurement sensor 30 described above changes in the opposite direction, having light quantity gas isosbestic points at the peak wavelengths (435 nm and 415 nm) of the absorption spectrum, according to the extent of the reaction with NO. For example, when the NO concentration in exhaled air introduced into the chamber 13 rises, the 415 nm light quantity goes up and the 435 nm light quantity goes down. On the other hand, when the NO concentration in the exhaled air introduced into the chamber 13 drops, the 415 nm light quantity goes down and the 435 nm light quantity goes up. In FIG. 3, the one-dot chain line is the transmission spectrum curve of a state in which almost all of the cobalt in the detection region is divalent, the broken line is the transmission spectrum curve of a state in which almost all of the cobalt in the detection region is trivalent, and the solid line is the transmission spectrum curve of a state in which divalent cobalt and trivalent cobalt are both present in substantially the same amounts in the detection region.

In this embodiment, thus detecting both the 415 nm light quantity and the 435 nm light quantity and outputting the differential removes common mode noise and increases the amount of change in the voltage value attributable to the NO concentration (the value of the differential output). Thus, compared to when just the 415 nm light quantity is detected and the NO concentration is measured, for example, smaller changes can be easily detected, which enhances the detection sensitivity.

The sensitivity correction processor 19 inputs the outputs from the current/voltage converting circuit 16d on the 415 nm light quantity detector 16 side and from the differential processor 18. The memory unit 20, which is used for storing information necessary for NO concentration measurement and correction, is connected to the sensitivity correction processor 19.

Calibration data for converting the value of the differential output from the differential processor 18 (voltage value) into a NO concentration value, and sensitivity correction information used for correction of the differential output based on the 415 nm light quantity (discussed in detail below) are pre-stored in the memory unit 20.

The above-mentioned calibration data is data (a mathematical formula or a graph) indicating the relation between a standard substance of known quantity, activity, etc., and the measured data with respect to it, and is used in quantitative experiments and testing to find the quantity or concentration of a substance, activity, or the like. Since there is nothing special about the calibration data used in this embodiment, it will not be described in detail here.

Information Stored in Memory Unit 20

Of the information stored in the memory unit 20, the sensitivity correction information used for correcting the differential output based on the 415 nm light quantity will be described below.

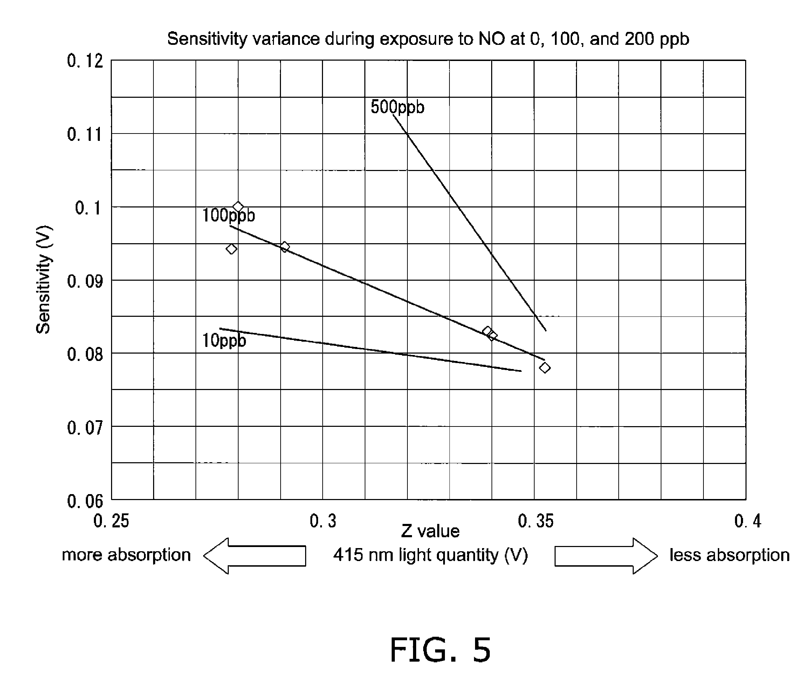

Specifically, there is a correlation between the amount of change in the differential output for a specific NO concentration from a plurality of NO concentration measurement sensors 30, and the amount of light with a wavelength (415 nm in this embodiment) indicating that the cobalt is in a divalent state during zero gas correction, and it was found that when this is put into a graph as shown in FIG. 5, if the points plotting the experimental results are shown as a straight line the least squares method, there will be a relatively strong correlation, and the correlation will be substantially linear. This correlation line is expressed by the following mathematical formula (1) when the NO concentration is 100 ppb, for example. The degree of this correlation was strong, at $R^2 = 0.9465$.

$$y = -0.2486x + 0.1668 \ ... \ (1)$$

The correlation lines shown in FIG. 5 are a graph of the correlation between the amount of change in the differential output at various NO concentrations and the 415 nm light quantity during zero gas correction with respect to a plurality of sensors when NO was introduced into the chamber 13 in concentrations of 10, 100, and 500 ppb following zero gas correction. These correlation lines show that regardless of the NO concentration, when the 415 nm light quantity is smaller, there is a rise in the amount of change in the differential output of the NO concentration measurement sensor 30, and when the 415 nm light quantity is larger, there is a decrease in the amount of change in the differential output of the NO concentration measurement sensor 30.

The following is a possible cause of this correlation.

When there is variance in the amount of supported porphyrin due to variance in the thickness of the base material at the portion of the NO concentration measurement sensor 30 where light is transmitted, the porphyrin solution concentration, the amount of porphyrin solution that is dripped, and so forth, saying that the 415 nm light quantity is large means that the amount of 415 nm light that is absorbed is small, and there is a reduction in the amount of change in the differential output because a relatively small amount of porphyrin has reacted with NO. Conversely, saying that the 415 nm light quantity is small means that the amount of 415 nm light that is absorbed is large, and there is an increase in the amount of change in the differential output because a relatively large amount of porphyrin has reacted with NO.

**[0046]** Accordingly, as shown in FIG. 5, even when NO is introduced in a given concentration (100 ppb), if the base material 31 is thick are the portion of the NO concentration measurement sensor 30 where the light is transmitted, and there is a large amount of supported porphyrin, then the NO detection sensitivity will be higher, and conversely, if the base material 31 is thin are the portion of the NO concentration measurement sensor 30 where the light is transmitted, and there is a small amount of supported porphyrin, then the NO detection sensitivity will end up being lower. Consequently, it is conceivable that variance in the detection sensitivity will occur from one NO concentration measurement sensor 30 to the next, due to variance in the amount of supported porphyrin as a result of the thickness of the base material 31, the extent to which the fibers are intertwined, and so forth from one NO concentration measurement sensor 30 to the next.

Also, experimental results revealed that the slope of the correlation lines shown in FIG. 5 varies with the NO concentration.

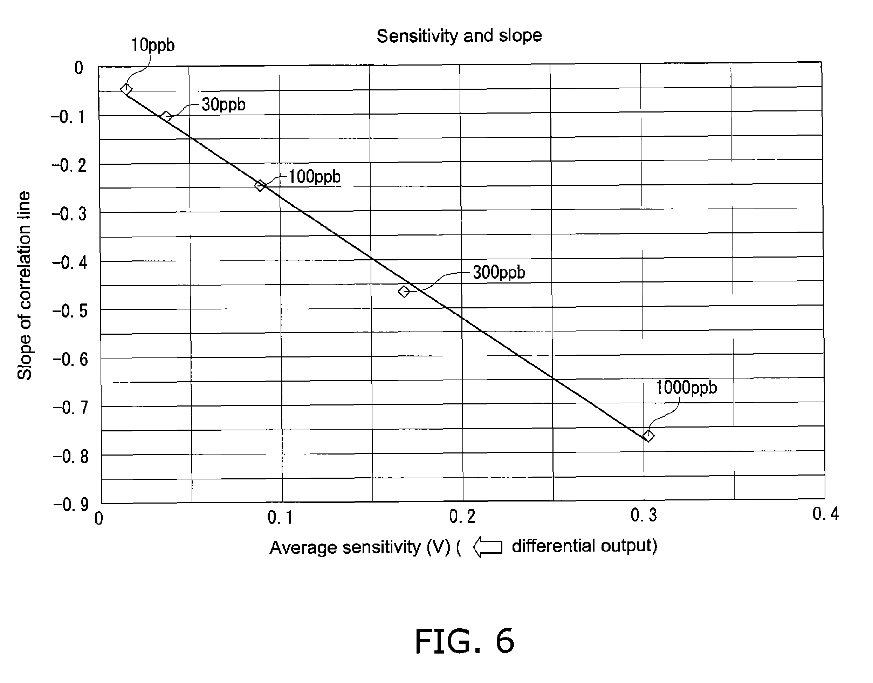

FIG. 6 is a graph of the relation between the amount of change in the differential output and the slope of the correlation line.

Here, the amount of change in the differential output on the horizontal axis of the graph in FIG. 6 is strongly correlated with NO concentration since the change increases when the NO concentration is higher and decreases when the NO concentration is lower. In this graph, the experimental results of finding the relation between the amount of change in the differential output at different NO concentrations (10, 30, 100, 300, and 1000 ppb) and the slope of correlation lines at various NO concentrations exhibits a strong correlation of $R^2 = 0.9968$, and when the correlation line is found by the method of least squares, the result is a straight line expressed by the following. More specifically, the graph in FIG. 6 shows that when the NO concentration is low, the slope of the correlation line is smaller, and when the NO concentration is high, the slope of the correlation line is larger.

$$y = -2.5152x - 0.0186 \ ... \ (2)$$

Thus, in actual NO concentration measurement, the sensitivity correction processor 19 that has obtained the differential output from the differential processor 18 can find the value of the slope of the correlation line on the vertical axis by using the above mathematical formula (2) or by plugging the value thereof into the numerical value on the horizontal axis on the basis of the graph in FIG. 6.

Processing to correct the NO concentration through the use of the above mathematical formulas (1) and (2) (or the graphs in FIGS. 5 and 6) will be discussed in detail below.

NO Concentration Measurement Sensor 30

The NO concentration measurement sensor 30 used in the nitrogen oxide concentration measurement device 10 of this embodiment is a transmission type of sensor that transmits light emitted from the LED light source 11, and the porphyrin shown in FIG. 2 (CoT(dtBu)PP) is supported at the portion where the light is transmitted.

As shown in FIG. 4, for example, the NO concentration measurement sensor 30 is manufactured by preparing a solution in which a powder of the porphyrin shown in FIG. 2 (CoT(dtBu)PP) is dissolved in a concentration of 5E-5 mol/L in chloroform (guaranteed reagent) or another such solvent, and dripping this solution in drops of 10 $\mu$L each from a drip nozzle with an inside diameter of 200 $\mu$m onto the base material 31 composed of paper (cellulose) or nonwoven cloth made of PET (polyethylene terephthalate) fibers.

The base material 31 can be, for example, a nonwoven cloth made from PET fibers, or a nonwoven cloth made from cellulose fibers (such as filter paper grade 514A, made by Advantec).

The liquid discharge apparatus used for dripping the porphyrin solution onto the base material 31 can be, for example, a Shot Master (with a height control device) made by Musashi Engineering.

In this embodiment, the NO concentration measurement sensor 30 is manufactured under the following conditions for the height of the drip nozzle, the discharge rate of the porphyrin, the discharge amount, and the like, so that the porphyrin will penetrate the base material 31 in an agglomerated state by about the same amount each time. This is because if the agglomerated state of the porphyrin changes every time, the degree of correlation will suffer and the sensitivity variance correction effect will decrease.

More specifically, the drip nozzle height was 0 to 5 $\mu$m, the discharge amount was 10 $\mu$L, the discharge speed was 20 seconds, the diameter of the drip nozzle was 200 mm, and the base material was paper (grade 514A).

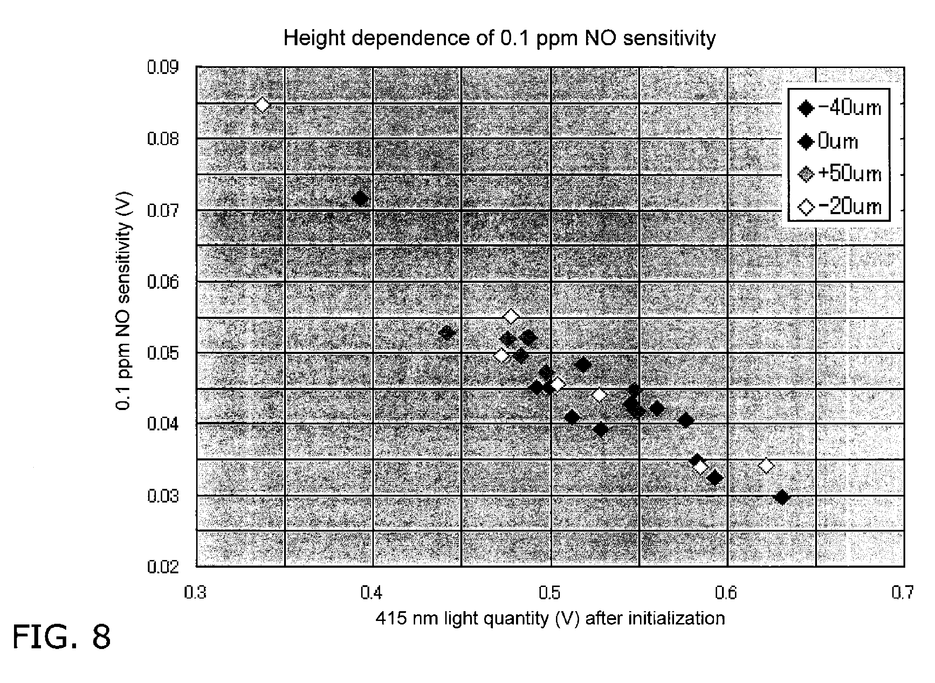

FIG. 8 shows the variance in NO sensitivity when the drip nozzle height was varied. In FIG. 8, the horizontal axis shows the 415 nm light quantity and the vertical axis shows the 100 ppb NO sensitivity. The NO sensitivity under a 100 ppb NO concentration environment is shown at different drip nozzle heights. The drip nozzle heights of -40 $\mu$m, 0 $\mu$m, +50 $\mu$m, and -20 $\mu$m shown in FIG. 8 are distances indicating the height from the surface of the base material 31 to the drip nozzle tip, and negative (-) values refer to a state in which the nozzle tip is recessed under the surface of the base material 31.

As can be seen from this graph, there is less variance at drip nozzle heights of 0 $\mu$m and -20 $\mu$m than at other heights.

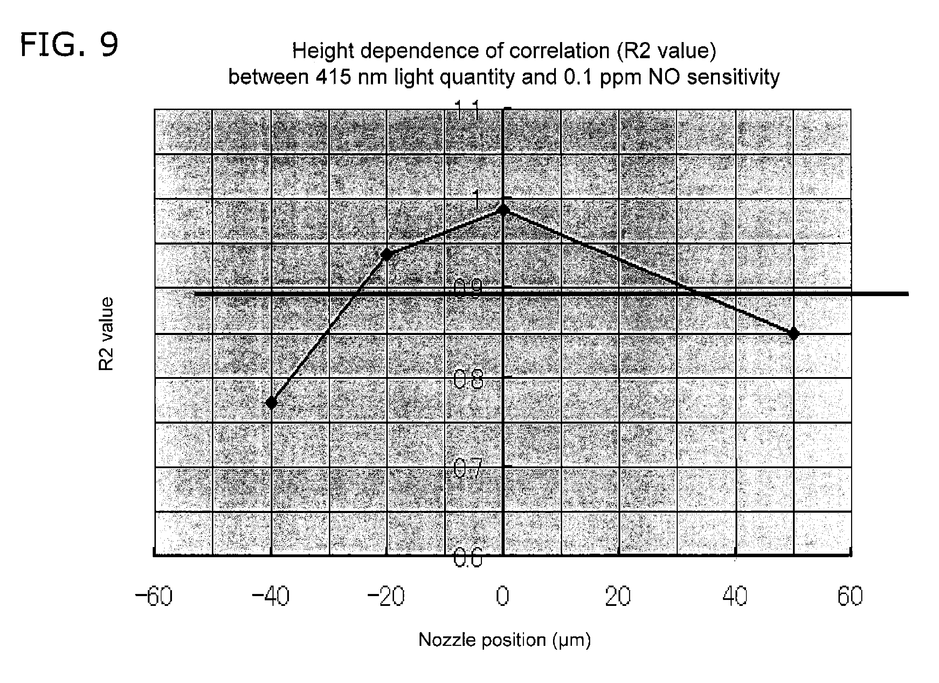

In FIG. 9, the nozzle position is shown on the horizontal axis, and the $R^2$ value representing the degree of correlation is shown on the vertical axis. This graph shows the height of correlation between the 415 nm light quantity and the NO sensitivity under an NO concentration environment of 100 ppb.

As can be seen from this graph, in order for $R^2$ to be 0.9 or more, the height of the drip nozzle is preferably from -25 to 25 $\mu$m. It was found experimentally that a good sensitivity variance correction effect can be obtained if the $R^2$ value is 0.9 or more.

Sensitivity variance due to changes in the agglomeration state of the porphyrin from one NO concentration measurement sensor 30 to the next can be minimized by manufacturing in which the porphyrin solution is dripped under the above conditions. Sensitivity variance can be kept to a minimum by combination with the following correction control.

Measurement of NO Concentration and Correction Control of Measurement Values

As shown in FIG. 1, with the nitrogen oxide concentration measurement device 10 in this embodiment, the change in the amount of light of a specific wavelength (415 or 435 nm) is detected, out of the transmitted light emitted from the LED light source 11 at the NO concentration measurement sensor 30 in a state in which exhaled air from an asthma patient or the like has been introduced into the chamber 13, inside of which the NO concentration measurement sensor 30 has been installed, and the differential output (NO concentration value) is corrected by utilizing the correlation lines shown in FIG. 5, using the differential output and the 415 nm light quantity.

The concentration of NO contained in exhaled air was actually measured using the above configuration, and the flow of control up to the correction of these measurement values will now be described in specific terms.

With the nitrogen oxide concentration measurement device 10 in this embodiment, first the 415 nm light quantity is detected by the 415 nm light quantity detector 16 out of the light transmitted by the portion of the NO concentration measurement sensor 30 where the porphyrin is supported, in a state in which correction-use zero gas has been introduced into the chamber 13, and this detection result is sent as a voltage value to the sensitivity correction processor 19.

Next, the differential processor 18 subtracts the voltage value indicating the 435 nm light quantity from the voltage value indicating the 415 nm light quantity, calculates the differential (differential output), and outputs this to the sensitivity correction processor 19.

Next, exhaled air is introduced into the chamber 13, and the 415 nm light quantity and the 435 nm light quantity are detected by the 415 nm light quantity detector 16 and the 435 nm light quantity detector 17, respectively, out of the light transmitted by the portion of the NO concentration measurement sensor 30 where the porphyrin is supported.

The 415 nm light quantity detector 16 and the 435 nm light quantity detector 17 output their detection results as voltage values to the differential processor 18, and the differential processor 18 subtracts the voltage value indicating the 435 nm light quantity from the voltage value indicating the 415 nm light quantity, calculates the differential (differential output), and outputs this to the sensitivity correction processor 19.

Next, the sensitivity correction processor 19 calculates the slope of the correlation lines shown in FIG. 5 on the basis of the value of the differential output (voltage value) received from the differential processor 18, and sensitivity correction information stored in the memory unit 20 (in this embodiment, the above mathematical formula (2)). More specifically, the sensitivity correction processor 19 plugs in the value of the differential output as the value of x in the above mathematical formula (2), and calculates the y value (the slope of the correlation lines in FIG. 5).

A mathematical formula may be used as in this embodiment as the correction sensitivity information for calculating the slope of the correlation line, or a table giving the relation between the differential output and the slope of the correlation line, or the graph shown in FIG. 6 can be used.

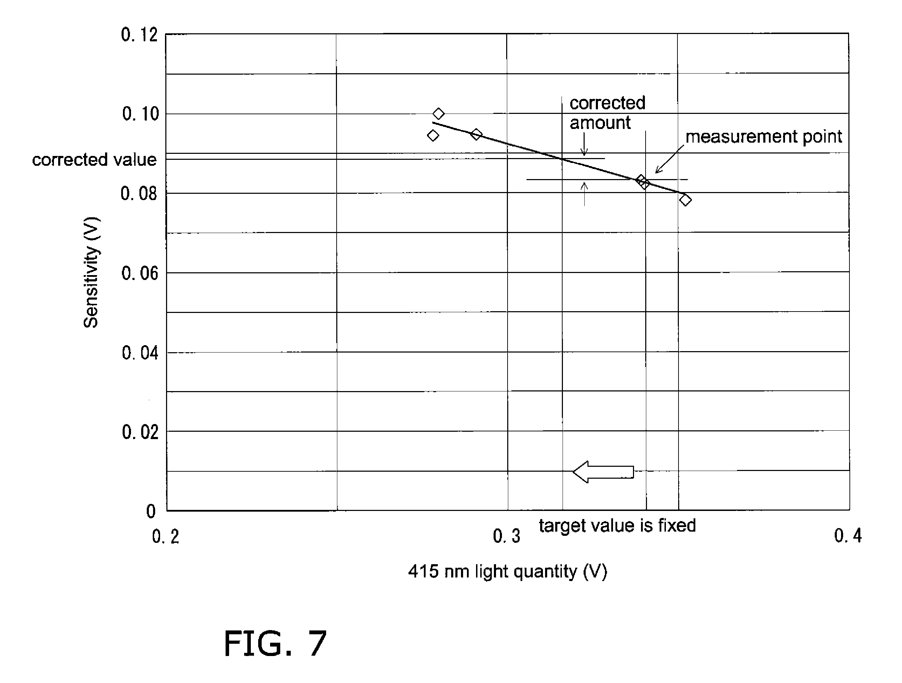

Next, the sensitivity correction processor 19 corrects the differential output on the basis of the voltage value indicating the 415 nm light quantity received from the current/voltage converting circuit 16d of the 415 nm light quantity detector 16 with respect to the correction-use zero gas, the preset reference value for the 415 nm light quantity shown in FIG. 7, and the y value (slope of the correlation line) calculated on the basis of the mathematical formula (2). The corrected value of the differential output is found from the following equation.

$$\text{(corrected value of differential output)} = \text{differential output} - \text{(slope of correlation line)} \times \text{(415 nm light quantity at zero gas calibration} - \text{415 nm light quantity reference value)}$$

FIG. 7 shows an example of a straight line having a slope of when the NO concentration is 100 ppb, but actually the slope varies with the NO concentration, so we shall assume that the differential output is corrected by utilizing a correlation line having a slope calculated from mathematical formula (2).

This correction processing will now be described through reference to a graph. As shown in FIG. 7, the measurement point is moved with respect to the preset reference value of the 415 nm light quantity along the correlation line, so that the measurement result is corrected as if the amount of absorption were not changing. In this embodiment, by way of example, the corrected value is calculated by plugging in 0.328 V, which corresponds approximately to the center of the correlation line shown in FIG. 7, for the value x in the mathematical formula as a target value, and the corrected value is calculated as the voltage value (approximately 0.881) indicating sensitivity after correction from the voltage value (approximately 0.831) prior to correction, which indicates the sensitivity at the measurement point.

Next, the sensitivity correction processor 19 converts the corrected voltage value 0.881 into an NO concentration using a calibration data curve, allowing a corrected NO concentration value to be obtained.

With the nitrogen oxide concentration measurement device 10 in this embodiment, variance in the detection sensitivity from one NO concentration measurement sensor 30 to the next, which is caused by variance in the amount of porphyrin absorbed at the NO concentration measurement sensor 30, can be suppressed by performing the correction control discussed above. That is, performing the above correction control allows constant measurement of the amount of porphyrin absorption, etc., at the NO concentration measurement sensor 30 to be carried out equivalently.

As a result, variance in sensitivity caused by variance in the amount of absorbed porphyrin from one NO concentration measurement sensor 30 to the next can be corrected, affording more accurate NO concentration measurement.

Other Embodiments

An embodiment of the present invention was described above, but the present invention is not limited to or by the above embodiment, and various modifications are possible without departing from the gist of the invention.

(A) In the above embodiment, an example was given in which the NO concentration was measured by using an NO concentration measurement sensor, in which is supported a porphyrin containing cobalt as its central metal, to detect the 415 nm light quantity and the 435 nm light quantity, but the present invention is not limited to this.

In the measurement of the NO concentration, rather than detecting light of two different wavelengths as above, the NO concentration can instead be measured by detecting just the change in the 415 nm light quantity, for example. However, in terms of being able to increase detection sensitivity with ease, it is preferable for there to be two light detectors (first and second light detectors), to detect the 415 nm light quantity and the 435 nm light quantity, and to use the difference between the two to measure the NO concentration, as in the above embodiment.

(B) In the above embodiment, an example was given in which the NO concentration was measured by using an NO concentration measurement sensor in which is supported a porphyrin containing cobalt as its central metal, but the present invention is not limited to this.

For example, an NO concentration measurement sensor in which is supported a porphyrin containing another metal as its central metal may be used instead.

Here again, the NO concentration can be measured by detecting the amount of change in light of a specific wavelength that varies upon reaction with NO.

(C) In the above embodiment, an example was given in which the NO concentration was measured by detecting transmitted light emitted at an NO concentration measurement sensor in which is supported a porphyrin containing cobalt as its central metal, but the present invention is not limited to this.

For example, it is also possible to use a sensor that includes a base material that reflects emitted light.

In this case, alumina, silicon, silicon carbide, gallium arsenide, ceramic, plastic, metal or another such base material that reflects light can be used as the base material of the sensor.

(D) In the above embodiment, an example was given of using an NO concentration measurement sensor in which porphyrin is supported on a base material 31 composed of paper or cellulose fibers, but the present invention is not limited to this.

For example, a detecting film that includes a porphyrin but has not base material may be used as the NO concentration measurement sensor. Alternatively, a sensor may be used in which the base material is a nonwoven cloth made of fibers other than cellulose, such as PET fibers.

(E) In the above embodiment, an example was given in which the NO concentration was measured using an NO concentration measurement sensor 30 containing porphyrin (CoT(dtBu)PP), but the present invention is not limited to this.

For example, even when using NO concentration measurement sensors including metal complexes of derivatives of porphyrin, the same effects as above can be obtained by measuring the amount of light of the peak wavelength in the absorption spectrum, which varies with reaction with NO.

Embodiment 2

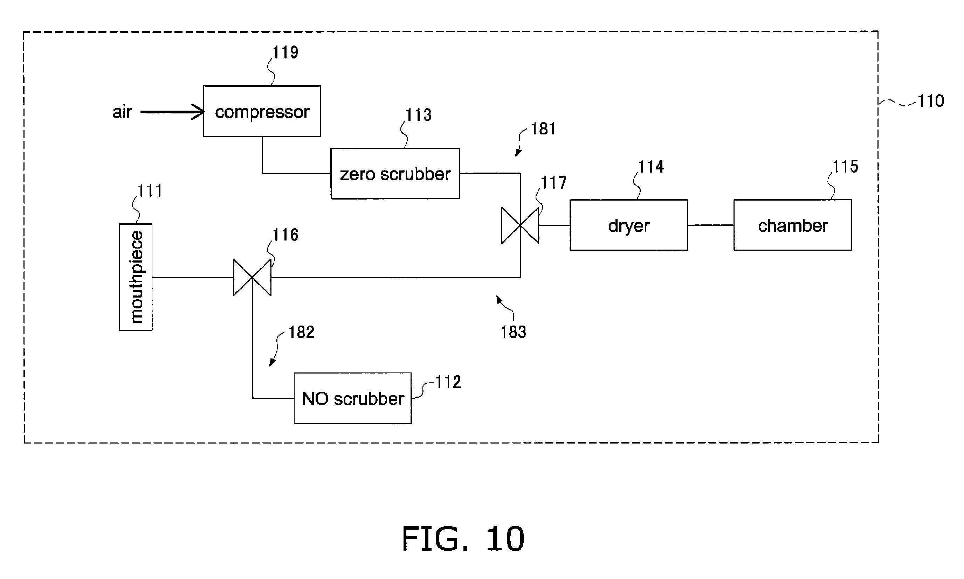

The nitrogen oxide concentration measurement device 110 pertaining to another embodiment of the present invention will now be described through reference to FIGS. 10 to 14.

Configuration of Nitrogen Oxide Concentration Measurement Device 110

The nitrogen oxide concentration measurement device 110 pertaining to this embodiment is an apparatus for measuring the concentration of nitric oxide (NO) in the exhaled air of asthma patients, etc. As discussed below, in this embodiment the NO concentration is measured in particular on the basis of the amount of transmitted light from an NO concentration measurement sensor 130. The NO concentration measurement sensor 130 will be described in detail below.

As shown in FIG. 10, the nitrogen oxide concentration measurement device 110 comprises a mouthpiece 111, an NO scrubber 112, a zero scrubber 113, a dryer 114, a chamber 115, valves 116 and 117, a compressor 119, and tubing to link the various units.

The tubing and the valves 116 and 117 form a first path 181, a second path 182, and a third path 183. The first path 181 leads from the compressor 119, through the zero scrubber 113 and the dryer 114, to the chamber 115. One end of the second path 182 is connected to the NO scrubber 112, and the other end is connected to the mouthpiece 111 and via the valve 116. The third path 183 leads from the mouthpiece 111, without passing through the zero scrubber 113, through the dryer 114, to the chamber 115.

Although not depicted, the nitrogen oxide concentration measurement device 110 may also comprise a flow gauge for measuring the flow or pressure of exhaled air and atmospheric air, a pressure gauge, a thermometer for sensing temperature, and so forth.

The mouthpiece 111 is formed so that the user can place it against his mouth and blow into it.

The NO scrubber 112 has a hole for taking in air. The NO scrubber 112 is configured so as to eliminate NO from the air by being equipped with activated carbon, potassium permanganate, or the like.

The zero scrubber 113 is configured so as to eliminate NO from exhaled air blown in from the mouthpiece 111, by being equipped with activated carbon, potassium permanganate, or the like. The NO scrubber 112 and the zero scrubber 113 may instead be constituted by a single scrubber.

The dryer 114 is equipped with silica gel, for example, and is configured so as to dehumidify the exhaled air.

The chamber 115 is a housing inside of which the NO concentration measuring sensor 130 is installed, and has a gas inlet 115a into which exhaled air or the like is introduced, and a gas outlet 115b for discharging the same.

The compressor 119 pumps air into the zero scrubber 113.

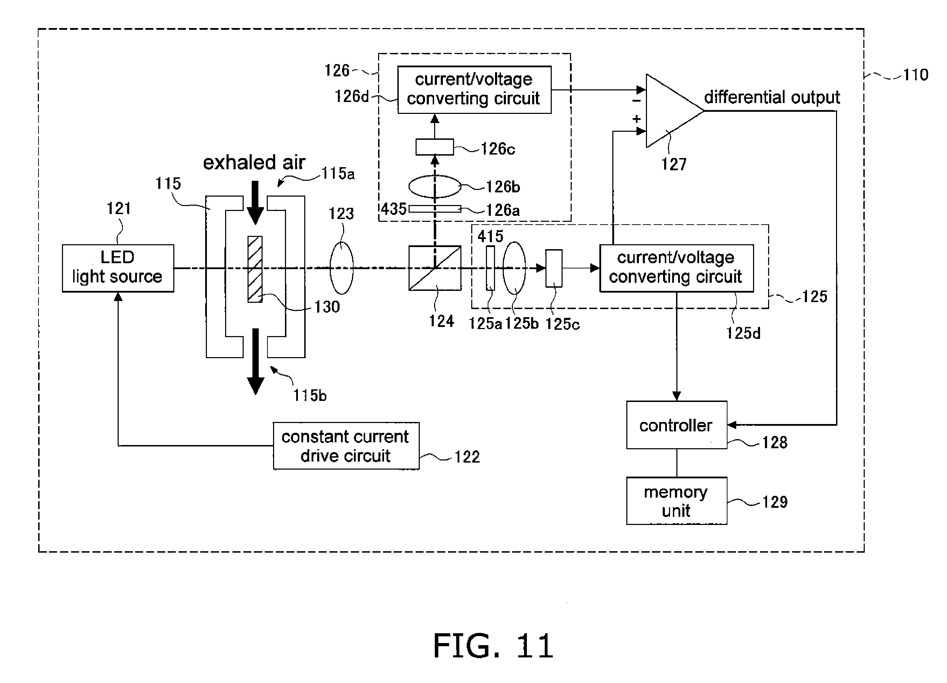

As shown in FIG. 11, the nitrogen oxide concentration measurement device 110 further comprises an LED light source 121, a constant current drive circuit 122, a lens 123, a dichroic mirror 124, a 415 nm transmitted light quantity detector 125, a 435 nm transmitted light quantity detector 126, a differential amplifier circuit 127, a controller 128, and a memory unit 129.

The LED light source 121 emits light under specific conditions onto the NO concentration measurement sensor 130 provided in the chamber 115.

The constant current drive circuit 122 adjusts the amount of light directed at the NO concentration measurement sensor 130 from the LED light source 121 by varying the current applied to the LED light source 121 under the control of the controller 128.

The lens 123 and the dichroic mirror 124 are disposed in that order between the chamber 115 and the 415 nm transmitted light quantity detector 125 and 435 nm transmitted light quantity detector 126. The dichroic mirror 124 transmits light near 415 nm out of all the light transmitted by the NO concentration measurement sensor 130 and received via the lens 123, and reflects light of other wavelengths.

The 415 nm transmitted light quantity detector 125 is disposed along the path of the light transmitted by the dichroic mirror 124. As shown in FIG. 10, the 415 nm transmitted light quantity detector 125 comprises a bandpass filter (BPF) 125a, a lens 125b, a photodiode (PD) 125c, and a current/voltage conversion circuit 125d.

The bandpass filter 125a is an optical filter that transmits only light near 415 nm. That is, the bandpass filter 125a separates the 415 nm light from the light transmitted by the dichroic mirror 124 and the NO concentration measurement sensor 130, and guides this light to the lens 125b.

The lens 125b guides the 415 nm light transmitted by the bandpass filter 125a to the photodiode 125c.

The photodiode 125c outputs a current corresponding to the amount of light received.

The current/voltage conversion circuit 125d converts the current coming from the photodiode 125c into voltage and outputs it to the differential amplifier circuit 127 and the controller 128.

With the above configuration, the 415 nm transmitted light quantity detector 125 takes out the 415 nm light from the light transmitted by the NO concentration measurement sensor 130, and detects the amount of this light.

The 435 nm transmitted light quantity detector 126 is disposed along the path of light transmitted by the NO concentration measurement sensor 130 and reflected by the dichroic mirror 124. As shown in FIG. 10, the 435 nm transmitted light quantity detector 126 comprises a bandpass filter 126a, a lens 126b, a photodiode 126c, and a current/voltage conversion circuit 126d.

The bandpass filter 126a transmits only light near 435 nm.

The lens 126b guides the light transmitted by the bandpass filter 126a to the photodiode 126c.

The photodiode 126c outputs a current corresponding to the amount of light received.

The current/voltage conversion circuit 126d converts the current coming from the photodiode 126c into voltage and outputs it to the differential amplifier circuit 127.

With the above configuration, the 435 nm transmitted light quantity detector 126 takes out the 435 nm light from the light transmitted by the NO concentration measurement sensor 130, and detects the amount of this light.

The differential amplifier circuit 127 outputs to the controller 128 the difference D (Vi - Vii) between the voltage value outputted from the 415 nm transmitted light quantity detector 125 (that is, the signal Vi) and the voltage value outputted from the 435 nm transmitted light quantity detector 126 (that is, the signal Vii).

The controller 128 controls the operation of the various units of the nitrogen oxide concentration measurement device 110, and also performs concentration calculation and the like.

As will be discussed below, data such as calibration curve selection-use tables, calibration curves, and so forth is stored in the memory unit 129.

The operation of the nitrogen oxide concentration measurement device 110 will be discussed below.

NO Concentration Measurement Sensor 130

The NO concentration measurement sensor 130 comprises a base material and a metal complex of porphyrin or a derivative thereof (hereinafter also referred to simply as "metal complex") that is supported on the base material.

The base material may be any material that allows light to pass through, including a reflective material such as alumina, or a woven cloth, nonwoven cloth, porous material, or the like made of glass fibers or polyester, polypropylene, cellulose, or another such polymer. Nonwoven cloth is a structure made up of fibers that are intertwined without being woven, and paper is also encompassed by the concept of nonwoven cloth in this Specification. An example of a polyester is PET (polyethylene terephthalate). The metal complex is preferably worked into the base material. For example, the base material may be seeped with the metal complex, or an NO concentration measurement sensor may be formed by a mixture of a base material and a metal complex.

The metal complex is one whose transmission spectrum (also called the absorption spectrum) changes when exposed to a nitrogen oxide.

Derivatives of porphyrin encompass substances in which a substituent has been bonded to a porphyrin skeleton. Examples of substituents include a hydrocarbon group such as alkyl group, an alkylene group, or an aryl group, which may be branched; an alkoxy group such as a methoxy group or an ethoxy group; a halogen group; a hydroxy group; an amino group; an imino group; a nitro group, and a carbonyl group.

Examples of the metal that forms a coordinate bond with the porphyrin include cobalt, iron, magnesium, nickel, zinc, and vanadium.

In this embodiment, the NO concentration measurement sensor 130 comprises a nonwoven cloth and a metal complex supported on the nonwoven cloth (that is, that impregnates the nonwoven cloth).

The NO concentration measurement sensor 130 also comprises as a metal complex the CoT(dtBu)PP shown in FIG. 2, that is, a cobalt complex of a porphyrin derivative having a tertiary butyl group (t-Bu) located at the 3,5-position of a phenyl group.

As shown in FIG. 3, the CoT(dtBu)PP has an absorption peak near 415 to 420 nm the valence of the cobalt is 2 (Co(II)). The valence of cobalt changes from divalent to trivalent (Co(III)) when exposed to NO. Co(III) has an absorption peak near 435 to 440 nm. When the NO concentration measurement sensor 130 comprising CoT(dtBu)PP is exposed to the gas to be detected, if there is a large change in the NO concentration in the detected gas, there will be a large change in the absorption spectrum, and if the change in NO concentration is small, the change in the absorption spectrum will also be small.

Thus, the NO concentration can be measured by comparing the amount of light of a specific wavelength transmitted from the NO concentration measurement sensor 130 prior to exposure to the detected gas, and the amount of light of the specific wavelength transmitted from the NO concentration measurement sensors 130 after exposure.

In this embodiment, as discussed below, the differences D1 and D2 between a signal corresponding to the 415 nm light quantity and a signal corresponding to the 435 nm light quantity are acquired before and after exposure of the NO concentration measurement sensor 130 to the detected gas. The NO concentration is found on the basis of the difference between these differences D1 and D2 (D2 - D1) and a calibration curve.

The wavelength of light used for detection can be varied according to the configuration (the types of substituents) and the metal elements of the porphyrin or a derivative thereof. That is, 415 nm is an example of a first wavelength, and 435 nm is an example of a second wavelength.

As discussed below, the nitrogen oxide concentration measurement device 110 in this embodiment initializes the NO concentration measurement sensor 130 to a cobalt(II) state before the measurement of nitrogen oxide concentration. That is, this initialization puts the NO concentration measurement sensor 130 in a state of not being exposed to the substance to be detected. In this embodiment, the central metal of the complex is cobalt, and as discussed above, the NO concentration is measured on the basis of a change in the valence of the cobalt from divalent to trivalent. However, even without being exposed to the detected gas, cobalt will change from Co(II) to Co(III) when the NO concentration measurement sensor 130 reacts with $O_2$, nitrogen oxides, or the like in the atmosphere. Such changes reduce the detection accuracy. Therefore, the cobalt is reduced to Co(II) by initialization before being exposed to the detected gas.

Initialization is carried out by applying heat or light to the NO concentration measurement sensor 130.

Operation of Nitrogen Oxide Concentration Measurement Device 110

(1) Exhaled Air Uptake

Uptake of the exhaled air of the user is as follows. Switching of the path by the valves 116 to 118 is performed under the control of the controller 128.

First, the first path 181 is opened up by the valves 116 and 117. That is, the compressor 119, the zero scrubber 113, and dryer 114 are connected. NO is removed by the zero scrubber 113 from the air pumped in by the compressor 119, dehumidified by the dryer 114, and then goes into the chamber 115. The NO concentration measurement sensor 130 is thus exposed to air from which NO has been removed.

Next, when the user inhales air through the mouthpiece 111 in a state in which the mouthpiece 111 and the NO scrubber 112 are connected by the valve 116, the air from which NO has been removed by the NO scrubber 112 is inhaled through the second path 182 and into the user.

Next, the third path 183 is opened up by the valves 116 and 117. When the user exhales into the mouthpiece 111 in this state, the exhaled air enters the chamber 115 via the dryer 114 and the mouthpiece 111. The exhaled air at this point does not pass through the zero scrubber 113, so NO is included in the exhaled air.

Air from which NO has been removed will hereinafter be referred to as "zero gas," while exhaled air blown through the mouthpiece 111 will be referred to simply as "exhaled air." The exhaled air corresponds to the gas to be measured, and zero gas is a calibration gas for calibrating to 0 ppb.

In other words, the third path 183 leading from the mouthpiece 111 to the chamber 115 without passing through the zero scrubber 113 is a measured gas exposure path along which the NO concentration measurement sensor 130 is exposed to the measured gas, while the first path 181 leading from the compressor 119 to the chamber 115 through the zero scrubber 113 is a zero gas exposure path along which the NO concentration measurement sensor 130 is exposed to zero gas.

(2) Concentration Measurement

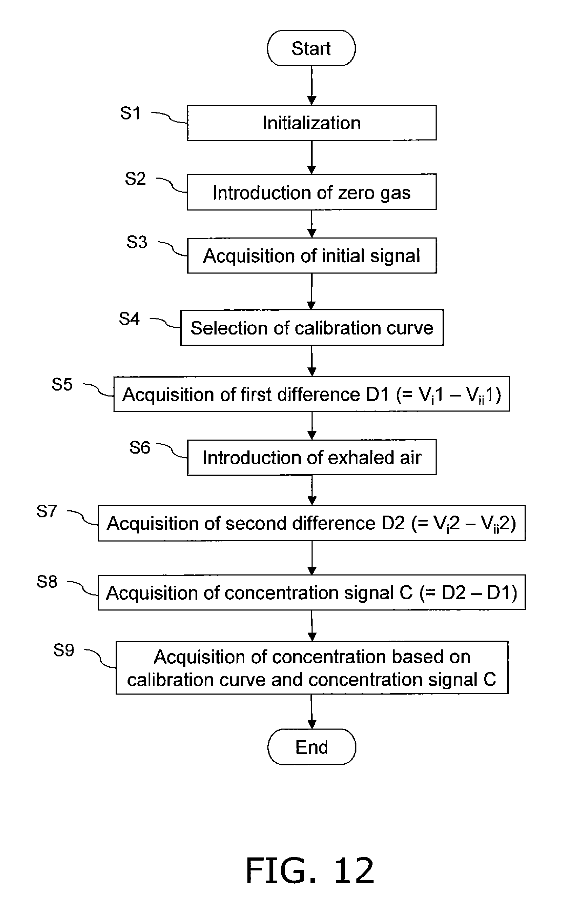

The operation in which NO concentration is measured with the nitrogen oxide concentration measurement device 110 will be described through reference to FIG. 12.

As shown in FIG. 12, initialization is performed in a state in which the NO concentration measurement sensor 130 is disposed in the chamber 115 (step S1).

Then, zero gas is introduced into the chamber 115 (step S2).

While the NO concentration measurement sensor 130 in the chamber 115 is being exposed to the zero gas, an initial signal V0 is acquired (step S3). More specifically, this entails the following. First, current is applied from the constant current drive circuit 122 to the LED light source 121, which causes the NO concentration measurement sensor 130 that is being exposed to the zero gas to be irradiated with light.

Of the irradiating light, the light transmitted by the NO concentration measurement sensor 130 goes through the lens 123 and is incident on the dichroic mirror 124. The incident light is separated by the 124 dichroic mirror into light near 415 nm and other light.

The transmitted by the dichroic mirror 124 (that is, light near 415 nm) is incident on the 415 nm transmitted light quantity detector 125, and of this, the 415 nm is transmitted by the bandpass filter 125a and guided through the lens 125b to the photodiode 125c. The current value outputted by the photodiode 125c according to the amount of light received is converted into a voltage value by the current/voltage conversion circuit 125d and outputted to the controller 128. The voltage signal thus generated is called the initial signal V0. The initial signal V0 corresponds to the 415 nm light quantity transmitted by the NO concentration measurement sensor 130 during zero gas exposure.

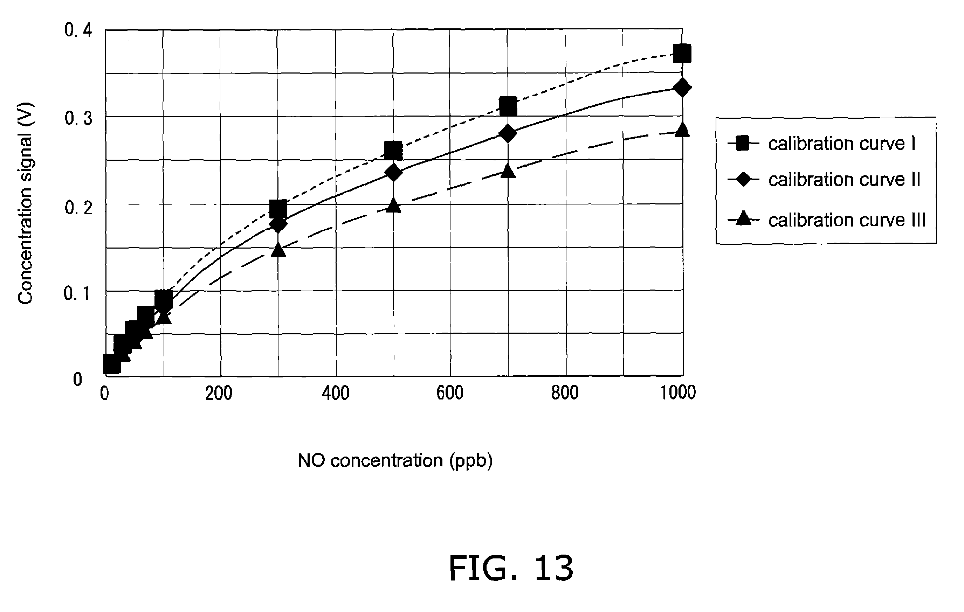

The controller 128 selects one calibration curve to be used in conversion to concentration (that is, in calibration) from among the calibration curves I to III (FIG. 13) stored in the memory unit 129 on the basis of the initial signal (step S4).

The calibration curves I to III are an example a plurality of sets of calibration information. The calibration curves I to III each have a different correlation to the NO concentration and the concentration signal. That is, in at least in part of the calibration curve I, the NO concentration corresponding to a given concentration signal is different from the NO concentration corresponding to that concentration signal in calibration curve II or III. The same applies to calibration curves II and III. The calibration information is not limited to three sets, and the number of sets may be changed. Calibration curves I to III can be found experimentally.

The memory unit 129 also stores at least one threshold used for calibration curve selection. In this embodiment, the memory unit 129 stores two threshold values, namely, 0.11 and 0.13. More specifically, the memory unit 129 stores the following calibration curve selection table.

Table 1

| Calibration curve | Initial signal (V) |
|---|---|
| I | less than 0.11 |
| II | at least 0.11 and less than 0.13 |
| III | at least 0.13 |

Specifically, calibration curve I is selected if the initial signal is less than 0.11 V, calibration curve II is selected if the initial signal is at least 0.11 V but less than 0.13, and calibration curve III is selected if the initial signal is at least 0.13 (step S4).

The initial signal V0 generated as above is outputted as a signal $V_i 1$ to the differential amplifier circuit 127.

Meanwhile, out of the light transmitted by the NO concentration measurement sensor 130, the light reflected by the dichroic mirror 124 (that is, transmitted light that is not near 415 nm) is incident on the bandpass filter 126a of the 435 nm transmitted light quantity detector 126. The 435 nm light transmitted by the bandpass filter 126a is guided through the lens 126b to the photodiode 126c. The current value outputted by the photodiode 126c according to the amount of light received is converted by the current/voltage conversion circuit 126d into a voltage value (that is, a signal $V_{ii}1$). The Signal $V_{ii}1$ is outputted to the differential amplifier circuit 127.

The differential amplifier circuit 127 outputs a first difference D1 (= $V_i1$ - $V_{ii}1$) to the controller 128 (step S5).

Next, exhaled air (that is, the measured gas) is introduced into the chamber 115 (step S6). As discussed above, the absorption spectrum of the metal complex contained by the NO concentration measurement sensor 130 fluctuates with the amount of NO in the exhaled air, with the 415 nm light quantity transmitted by the NO concentration measurement sensor 130 increasing, and the 435 nm light quantity transmitted by the NO concentration measurement sensor 130 decreasing. The amount of change in the 415 nm light quantity and the 435 nm light quantity transmitted by the NO concentration measurement sensor 130 is a function of the amount of change in the NO concentration from the zero gas to the measured gas.

Next, a second difference D2 ($V_i2$ - $V_{ii}2$) is acquired (step S7). More specifically, the signal $V_i2$, which is the voltage corresponding to the 415 nm light quantity transmitted by the NO concentration measurement sensor 130 with respect to the NO concentration measurement sensor 130 exposed to exhaled air, is acquired by the 415 nm transmitted light quantity detector 125, while the signal $V_{ii}2$, which is the voltage corresponding to the 435 nm light quantity transmitted by the NO concentration measurement sensor 130, is acquired by 435 nm transmitted light quantity detector 126. The differential amplifier circuit 127 outputs the second difference D2 ($V_i2$ - $V_{ii}2$) to the controller 128.

Then, the controller 128 acquires a concentration signal C by acquiring the difference (D2 - D1) between the first difference D1 and the second difference D2 (step S8).

The controller 128 finds the NO concentration (ppb) corresponding to the concentration signal C thus obtained, from the calibration curve selected in step S4 (step S9).

As described above, the controller 128 functions as a calibration information selector and a concentration determination unit.

Effect

Variance in the amount of metal complex and so forth between individual NO concentration measurement sensors 130 causes variance in sensitivity to NO (the amount of change in the amount of transmitted light with respect to the amount of change in the NO concentration). This variance appears in the variance in the initial signal. Thus, measurement error caused by variance in the sensitivity of the NO concentration measurement sensor 130 can be suppressed by selecting a calibration curve based on the initial signal.

In particular, if the NO concentration measurement sensor 130 comprises a base material into which the metal complex seeps, such as woven cloth, nonwoven cloth, or a porous material, variance in sensitivity tends to occur even though manufacture is performed under the same conditions. As a result, even though the exhaled air contains NO in the same concentration, there is the risk that different concentration values will be outputted as the measurement result due to sensitivity variance between NO concentration measurement sensors.

Also, with a NO concentration measurement sensor such as this, it is extremely difficult to support the metal complex uniformly, and even if it could be supported uniformly, variance in the thickness or basis weight of the base material (such as paper) may cause inplane variance in the impregnation amount of the metal complex in the NO concentration measurement sensor, making it more likely that variance will occur in sensitivity from one NO concentration measurement sensor to the next.

Variance in the sensitivity of an NO concentration measurement sensor such as this correlates with the amount of light transmitted by the NO concentration measurement sensor prior to exposure to NO. Specifically, the smaller is the amount of light transmitted prior to exposure to NO (that is, the lower is the initial signal strength), the higher is the sensitivity (that is, the greater is the amount of change in the amount of transmitted light at a given NO concentration). The larger is the amount of light transmitted prior to exposure to NO, the lower is the sensitivity (that is, the smaller is the amount of change in the amount of transmitted light at a give NO concentration).

If concentration were to be calculated with a given calibration curve using an NO concentration measurement sensor with a different sensitivity, there would be more variance in the concentration measurement result.

The nitrogen oxide concentration measurement device 110 in this embodiment can suppress variance in the measurement result by selecting a different calibration curve according to the initial signal strength.

Other Embodiments

(1) The initial signal may be obtained from light having a wavelength other than 415 nm.

(2) The base material NO concentration measurement sensor may be configured so as to reflect light, and the nitrogen oxide concentration measurement device may be configured so that various signals are obtained by detecting the amount of reflected light, rather than the amount of light transmitted from the NO concentration measurement sensor. In this case, alumina, silicon, silicon carbide, gallium arsenide, ceramic, plastic, metal, or another such base material that reflects light can be used as the base material of the sensor. Furthermore, the base material is not an essential unit.

(3) In the above embodiment, concentration was measured on the basis of the change in the amount of light of two different wavelengths (415 and 435 nm). However, the nitrogen oxide concentration measurement device may be configured such that concentration is measured on the basis of just the change in the amount of light of a single wavelength. That is, in the above embodiment, the 415 nm transmitted light quantity detector 125 and the 435 nm transmitted light quantity detector 126 were provided as light detectors, but the present invention is not limited to this.

(4) The calibration curves I to III are an example of calibration information. Four or more types of calibration information may be prepared, and the number of thresholds used for selecting the calibration curve can also be changed to match the number of types of calibration information. It is believed that using more thresholds and types of calibration information will afford more accurate measurement.

(5) In the above embodiment, the LED light source 121 was provided as a light irradiation unit, but an LED light source having a plurality of wavelength peaks (415 and 435 nm), or a plurality of LED light sources corresponding to different wavelengths may be provided.

Experimental Examples

(a) Manufacture of NO Concentration Measurement Sensor 130

FIG. 4 shows some of the steps of manufacturing the NO concentration measurement sensor 130 in this experimental example.

Grade 514A filter paper made by Advantec (nonwoven cloth of cellulose fibers, was cut with scissors to a size of 10 x 15 mm, dried at 130°C for 1.5 hours, and used as the base material 31.

A CoT(dtBu)PP powder was dissolved in chloroform (guaranteed reagent) to obtain a metal complex solution with a concentration of 5E-5 mol/L.

Using a Shot Master (made by Musashi Engineering, equipped with height control device), the metal complex solution was dripped onto the base material 31 at a discharge amount of 10 $\mu$L, a discharge rate of 20 seconds, and a drip nozzle diameter of 200 mm.

An NO concentration measurement sensor was produced by varying the drip nozzle height (the height from the surface of the base material 31 to the drip nozzle) in order to optimize the height of the drip nozzle. Using the NO concentration measurement sensors thus obtained, the sensitivity of the various NO concentration measurement sensors was compared for a gas with a known NO concentration, whereupon the inventors discovered that a drip nozzle height of from -25 $\mu$m to 25 $\mu$m is preferable to suppress variance in the sensitivity of an NO concentration measurement sensor.

Three NO concentration measurement sensors A to C manufactured at a nozzle height of 0 $\mu$m were used in the following experiment.

(b) Initialization and NO Concentration Measurement

An experiment was conducted using the following gas cylinders.
gas cylinder 001: compressed air
gas cylinder 002: 2.038 ppm NO base gas $N_2$
gas cylinder 003: 0.206 ppm NO base gas $N_2$

Each of the NO concentration measurement sensors manufactured as above was put in the chamber, and initialization with light was performed while compressed air was pumped from the gas cylinder 001 into the chamber at a flow rate of 100 mL per minute.

Upon completion of this initialization, the flow of compressed air from the gas cylinder 001 was raised to 2850 mL/min, and the air was introduced into the chamber as zero gas. A signal indicating the 415 nm light quantity here was obtained as the initial signal.

40 seconds after the start of the application of the measurement current, approximately 2 ppm of NO was supplied from the gas cylinder 002 and added to and mixed with the introduced zero gas for 15 seconds at 150 mL/min, which produced a (+)NO gas with a test NO concentration of 100 ppb, and this was pumped into the chamber at a total of 3 L/min. "(+)NO gas" is a gas containing NO, and in this experimental example it is the measured gas used as simulated exhaled air.

Over a total period of 35 seconds, comprising the 5 seconds in which the zero gas was introduced immediately before the introduction of the (+)NO gas, and the 15 seconds of zero gas introduction during and after the introduction of (+)NO gas, the 415 and 435 nm transmitted light quantities were measured every 0.2 second, the first difference D1 (= $V_i1-V_{ii}1$) and the second difference D2 ($V_i2 - V_{ii}2$) were acquired, and the difference between these the first difference D1 and the second difference D2 (D2 - D1) was acquired, which gave a concentration signal.

Further, the mix ratio of the compressed air and NO was varied, the NO concentration was adjusted to 100, 300, 500, 700, and 1000 ppb at a total flow rate of 3L/min, and each gas was pumped into the chamber as the (+)NO gas. Also, the mix ratio of the compressed air and NO was similarly varied using the gas cylinders 003 and 001, and (+)NO gas having an NO concentration of 70, 10, 30, or 50 ppb was prepared and fed into the chamber so that the total flow rate would be 3L/min.

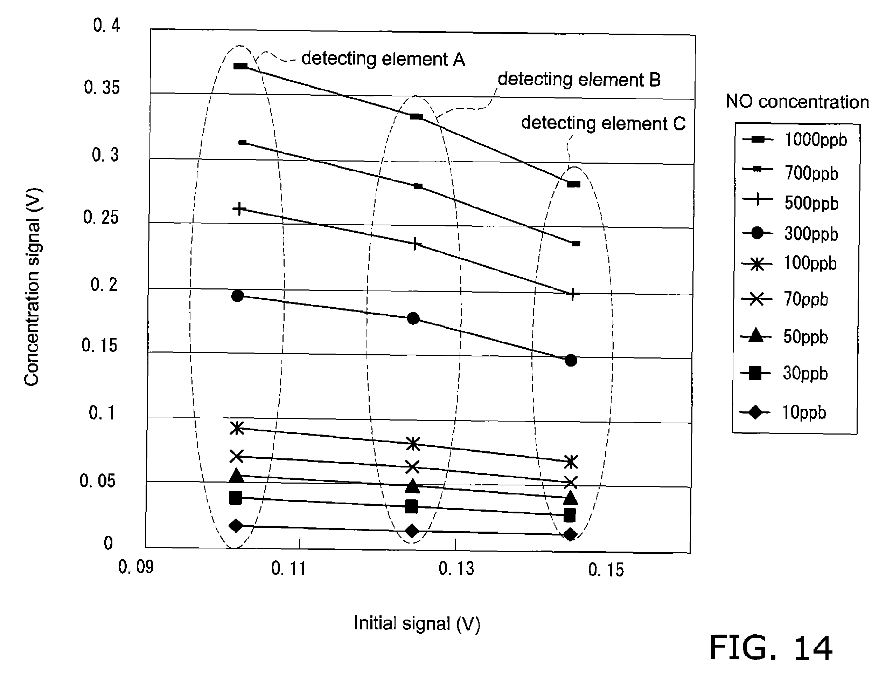

FIG. 14 is a graph showing the relation between the initial signal and the concentration signal obtained with each NO concentration measurement sensor. As shown in FIG. 14, it was found that the slope increases as the actual NO concentration rises. That is, it was found that accurate concentration measurement is difficult with a single calibration curve.

In view of this, the NO concentration was calculated on the basis of the calibration curve selection table, using the concentration signal of the NO concentration measurement sensor A as calibration curve I, the concentration signal of the NO concentration measurement sensor B as calibration curve II, and the concentration signal of the NO concentration measurement sensor C as calibration curve III.

Along with the three NO concentration measurement sensors A to C discussed above, an experiment was conducted on a total of 15 NO concentration measurement sensors. As a result, when the concentration was measured based on a single calibration curve, the coefficient of variation (CV) in concentration was 6% or more, but when the concentration was measured using three different calibration curves, the CV value was kept low, to 4% or less.

Embodiment 3

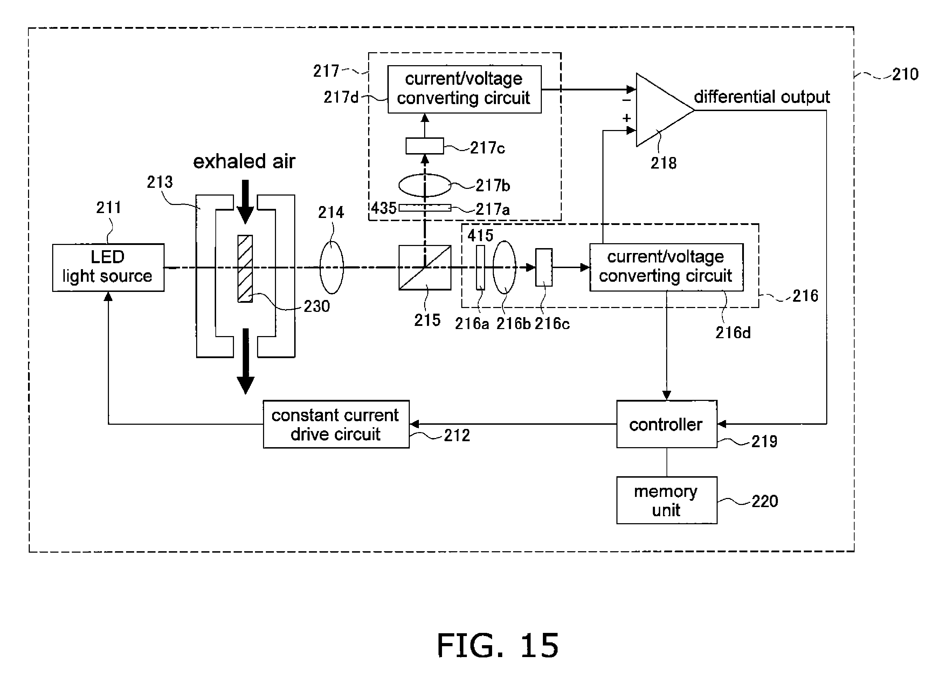

The nitrogen oxide concentration measuring device 210 pertaining to yet another embodiment of the present invention will now be described through reference to FIGS. 15 to 19.

In this embodiment, the output value from the current/voltage conversion circuit 216d shown in FIG. 15 refers to a voltage value (V) corresponding to the amount of light detected with a wavelength of 415 nm, and the amount of change in the output value from a differential processor 218 with respect to a specific amount of change in NO concentration refers to a voltage value (V) corresponding to sensitivity.

Configuration of Nitrogen Oxide Concentration Measuring Device 210

As shown in FIG. 15, the nitrogen oxide concentration measuring device 210 pertaining to this embodiment is a device that introduces exhaled air into a chamber 213 in which a nitric oxide (NO) (nitrogen oxide) concentration measurement sensor 230 is installed, detects the amount of light of a peak wavelength of the absorption spectrum that varies with the degree of reaction between NO and porphyrin (CoT(dtBu)PP) supported on the NO concentration measurement sensor 230 (see FIG. 2), and measures the concentration in which NO is contained in the exhaled air an asthma patient or the like. As shown in FIG. 15, the nitrogen oxide concentration measuring device 210 comprises an LED light source 211, a constant current drive circuit 212, the chamber 213, a lens 214, a dichroic mirror 215, a 415 nm light quantity detector (light detector, first detector) 216, a 435 nm light quantity detector (light detector, second detector) 217, a differential processor 218, a controller 219, and a memory unit 220.

The LED light source 211 irradiates the NO concentration measurement sensor 230 provided inside the chamber 213 with light under specific conditions.

The constant current drive circuit 212 is a circuit for determining the current applied to the LED light source 211, and adjusts the amount of light emitted from the LED light source 211 (hereinafter referred to as LED light quantity) by varying the current value to be applied. The constant current drive circuit 212 is connected to the controller 219, and the measurement current is adjusted by feedback control of the amount of light detected with a wavelength of 415 nm (discussed below).

In this embodiment, the constant current drive circuit 212 applies initialization current of 200 mA to the LED light source 211 during initialization of the NO concentration measurement sensor 230, and applies a measurement current of 25 mA upon initial application in the measurement of NO concentration. In this embodiment, initialization refers to a process at the stage of manufacturing the NO concentration measurement sensor 230, in which a porphyrin solution is dripped onto a base material 31 (see FIG. 4), after which the oxygen coordinate bonded to trivalent cobalt, which is the central metal of the porphyrin, is reduced to divalent. The NO concentration measurement sensor 230 on which porphyrin is supported will be described in detail at a later stage.

The chamber 213 is a housing inside of which the NO concentration measuring sensor 230 is installed, and has a gas inlet 213a into which exhaled air or the like is introduced, and a gas outlet 213b for discharging the same.

The light that has been emitted from the LED light source 211 and transmitted from the NO concentration measurement sensor 230 installed in the chamber 213 is guided by the lens 214 to the dichroic mirror 215, which is disposed at a later stage.

The dichroic mirror 215 transmits light near a specific wavelength (415 nm in this embodiment) out of the light transmitted by the NO concentration measurement sensor 230 and received via the lens 214, and reflects light of other wavelengths. This allows just the light of a specific wavelength (415 nm) to be taken out of the transmitted light.

The 415 nm light quantity detector 216 takes out light with a wavelength of 415 nm from the light transmitted by the dichroic mirror 215, and detects the amount of this light (hereinafter referred to as the 415 nm light quantity). As shown in FIG. 15, the 415 nm light quantity detector 216 has a bandpass filter (BPF) 216a, a lens 216b, a photodiode (PD) 216c, and the current/voltage converting circuit 216d.

The bandpass filter 216a transmits light with a wavelength near 415 nm out of the light transmitted by the dichroic mirror 215.

The lens 216b guides the light near 415 nm transmitted by the bandpass filter 216a to the photodiode 216c disposed at a later stage.

The photodiode 216c converts the light near 415 nm coming from the lens 216b into a current signal and outputs this signal.

The current/voltage converting circuit 216d converts the electrical signal coming from the photodiode 216c from a current value into a voltage value, and outputs the result to differential processor 218 and the controller 219.

The 435 nm light quantity detector 217 takes out the light with a wavelength near 435 nm reflected at the dichroic mirror 215, and detects the amount of this light (hereinafter referred to as the 435 nm light quantity). As shown in FIG. 15, the 435 nm light quantity detector 217 has a bandpass filter (BPF) 217a, a lens 217b, a photodiode (PD) 217c, and a current/voltage converting circuit 217d.

The bandpass filter 217a transmits the light with a wavelength near 415 nm reflected at the dichroic mirror 215.

The lens 217b guides the light near 415 nm transmitted by the bandpass filter 217a to the photodiode 217c disposed at a later stage.

The photodiode 217c converts the light near 415 nm coming from the lens 217b into a current signal and outputs this signal.

The current/voltage converting circuit 217d converts the electrical signal coming from the photodiode 217c from a current value into a voltage value, and outputs the result to the differential processor 218 and the controller 219.

The differential processor 218 outputs the difference between the 415 nm light quantity and the 435 nm light quantity that were converted into voltage values at the current/voltage converting circuit 216d on the 415 nm light quantity detector 216 side and the current/voltage converting circuit 217d on the 435 nm light quantity detector 217 side.

Here, as shown in FIG. 3, the porphyrin supported on the NO concentration measurement sensor 230 described above changes in the opposite direction, having light quantity gas isosbestic points at the peak wavelengths (435 nm and 415 nm) of the absorption spectrum, according to the extent of the reaction with NO. For example, when the NO concentration in exhaled air introduced into the chamber 213 rises, the 415 nm light quantity goes up and the 435 nm light quantity goes down. On the other hand, when the NO concentration in the exhaled air introduced into the chamber 213 drops, the 415 nm light quantity goes down and the 435 nm light quantity goes up. In FIG. 3, the one-dot chain line is the transmission spectrum curve for divalent cobalt, the broken line is the transmission spectrum curve for trivalent cobalt, and the solid line is the spectrum when divalent cobalt and trivalent cobalt are mixed.

In this embodiment, thus detecting both the 415 nm light quantity and the 435 nm light quantity and outputting the differential increases the amount of change in the voltage value attributable to the NO concentration (the value of the differential output). Thus, compared to when just the 415 nm light quantity is detected and the NO concentration is measured, for example, smaller changes can be easily detected, which enhances the detection sensitivity.

The controller 219 inputs the outputs from the current/voltage converting circuit 216d on the 415 nm light quantity detector 216 side and from the differential processor 218. The controller 219 is connected to the memory unit 220, which stores information required for NO concentration measurement (such as mathematical formulas and calibration curves). The controller 219 is also connected to the constant current drive circuit 212, which adjusts the LED light quantity of the LED light source 211, and performs feedback control based on the 415 nm light quantity (discussed below).

The memory unit 220 stores mathematical formulas (or calibration curves) used for converting the value (voltage value) of the differential output from the differential processor 218 into an NO concentration value. Consequently, a voltage value can be converted into a concentration value, and the NO concentration value can be calculated, by plugging the differential output of the 415 nm light quantity and the 435 nm light quantity into a mathematical formula or the like stored in the memory unit 220.

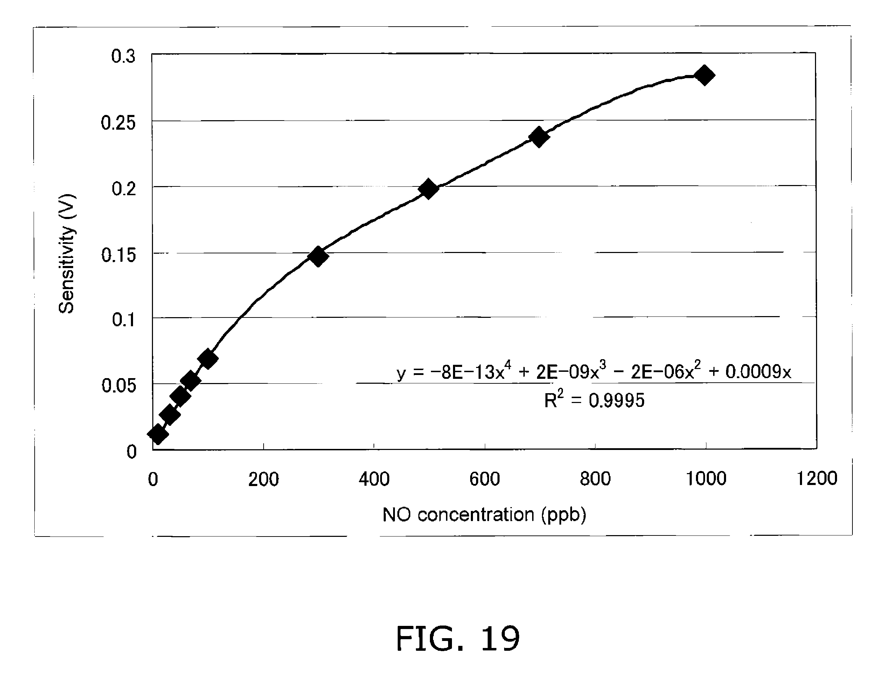

Here, the above-mentioned calibration curve is a graph of the relation between a standard substance of known quantity, activity, etc., and the measured data with respect to it, and is used in quantitative experiments and testing to find the quantity or concentration of a substance, activity, or the like. The calibration curve used in this embodiment is a graph produced by acquiring the same data using a plurality of NO concentration measurement sensors 230 at the point when conditions have been determined, and using the average value thereof (see FIG. 1, for example). This calibration curve will appear as different graphs depending on the base material and porphyrin concentrations, the conditions for dripping the porphyrin solution, and other conditions. FIG. 19 is merely an example of this.

NO Concentration Measurement Sensor 230

The NO concentration measurement sensor 230 used in the nitrogen oxide concentration measurement device 210 of this embodiment is a transmission type of sensor that transmits light emitted from the LED light source 211, and the porphyrin shown in FIG. 2 (CoT(dtBu)PP) is supported at the portion where the light is transmitted.

As shown in FIG. 4, for example, the NO concentration measurement sensor 230 is manufactured by preparing a solution in which a powder of the porphyrin shown in FIG. 2 (CoT(dtBu)PP) is dissolved in a concentration of 5E-5 mol/L in chloroform (guaranteed reagent) or another such solvent, and dripping this solution in drops of 0.5 to 30 μL each from a drip nozzle with an inside diameter of from 50 μm to 1 mm onto a base material 31 composed of paper (cellulose) or nonwoven cloth made of PET (polyethylene terephthalate) fibers.

The concentration of the porphyrin solution is determined by the amount of light absorption, which varies with the material, thickness, basis weight, and so forth of the sensor base material, and is the concentration at which the amount of absorption of 415 or 435 nm light will be from 30 to 80%. The production cost will go up if the nozzle diameter is less than 50 μm (inside diameter), and depending on the drip time and drip quantity, the internal pressure may rise, resulting in liquid leakage. If the diameter is over 1 mm, there is the risk of unwanted dripping or a change in concentration due to drying of the solvent.

The base material 31 can be, for example, a nonwoven cloth made from PET fibers, or a nonwoven cloth made from cellulose fibers (such as filter paper grade 514A, made by Advantec).

The liquid discharge apparatus used for dripping the porphyrin solution onto the base material 31 can be, for example, a Shot Master (with a height control device) made by Musashi Engineering.

In this embodiment, the NO concentration measurement sensor 230 is manufactured under the following conditions for the height of the drip nozzle, the discharge rate of the porphyrin, the discharge amount, and the like, so that the porphyrin will penetrate the base material 31 uniformly.

More specifically, the drip nozzle height was 5 μm, the discharge amount was 10 μL, the discharge speed was 20 seconds, the diameter of the drip nozzle was 200 mm, and the base material was paper (filter paper grade 514A, made by Advantec).

FIG. 8 shows the variance in NO sensitivity when the drip nozzle height was varied. The horizontal axis shows the 415 nm light quantity and the vertical axis shows the 100 ppb NO sensitivity. The NO sensitivity under a 100 ppb NO concentration environment is shown at different drip nozzle heights. The drip nozzle heights of -40 μm, 0 μm, +50 μm, and -20 μm shown in FIG. 8 are distances indicating the height from the surface of the base material 31 to the drip nozzle tip, and negative (-) values refer to a state in which the nozzle tip is recessed under the surface of the base material 31.

As can be seen from this graph, there is less variance at drip nozzle heights of 0 μm and -20 μm than at other heights.

In FIG. 9, the nozzle position is shown on the horizontal axis, and the $R^2$ value representing the degree of variance is shown on the vertical axis. This graph shows the correlation between the 415 nm light quantity and the NO sensitivity under an NO concentration environment of 100 ppb.

As can be seen from this graph, in order for $R^2$ to be 0.9 or more, the height of the drip nozzle is preferably from -25 to 25 μm.

Sensitivity variance from one NO concentration measurement sensor 230 to the next can be minimized by manufacturing in which the porphyrin solution is dripped under the above conditions. Sensitivity variance can be kept to a minimum by combination with the following correction control.

Measurement of NO Concentration and Correction Control of Measurement Values

As shown in FIG. 15, with the nitrogen oxide concentration measurement device 210 in this embodiment, the amount of light of a specific wavelength (415 or 435 nm) is detected, out of the transmitted light emitted from the LED light source 211 at the NO concentration measurement sensor 230 in a state in which exhaled air from an asthma patient or the like has been introduced into the chamber 13, inside of which the NO concentration measurement sensor 230 has been installed, and the NO concentration value calculated using the voltage value of the differential output thereof, and mathematical formulas and calibration curves stored in the memory unit 220.

Also, in this embodiment, in order to suppress the decrease in measurement accuracy caused by aging and the temperature characteristics of the LED light source 211 and the sensitivity variance of the NO concentration measurement sensor 230, the controller 219 performs feedback control to adjust the LED light quantity emitted from the LED light source 211 so that the 415 nm light quantity (voltage value) at the introduction of the initial NO concentration gas outputted from the 415 nm light quantity detector 216 will be constant.

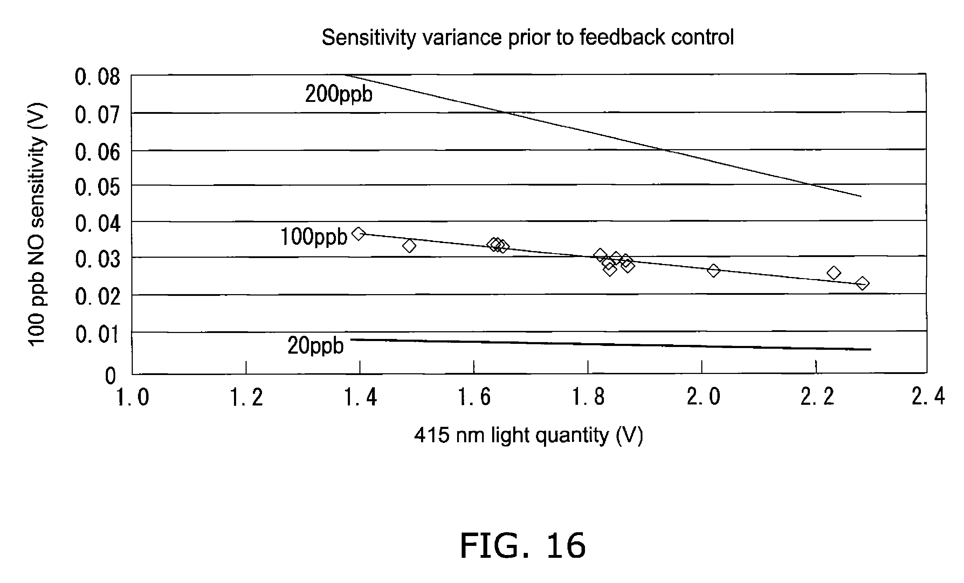

Here, when a plurality of NO concentration measurement sensors 230 are irradiated with the same LED light quantity from the LED light source 211 under the introduction of gas with NO concentrations of 20, 100, and 200 ppb, and the 415 nm light quantity is measured, for example, the NO detection sensitivity prior to feedback control for each NO concentration measurement sensor 230 can be seen from the experimental results to have a large amount of variance, regardless of the NO concentration, as shown in FIG. 16. More specifically, at an NO concentration of 100 ppb, for example, it can be seen that the 415 nm light quantity varies over a range of 0.023 to 0.036 V for NO detection sensitivity from approximately 1.4 V to approximately 2.3 V.

With the experiment results shown in FIG. 16, at an NO concentration of 100 ppb, the CV value, which indicates the extent (ratio) to which the sensitivity of 15 NO concentration measurement sensors 230 varied with respect to the average value, was 6.43%. As an  example, FIG. 16 shows a graph of correlation lines indicating the relation between the NO detection sensitivity and the 415 nm light quantity when the NO concentration was 20 and 200 ppb. As shown in FIG. 16, it can be seen that when the NO concentration changes, the slope of the correlation line also changes.

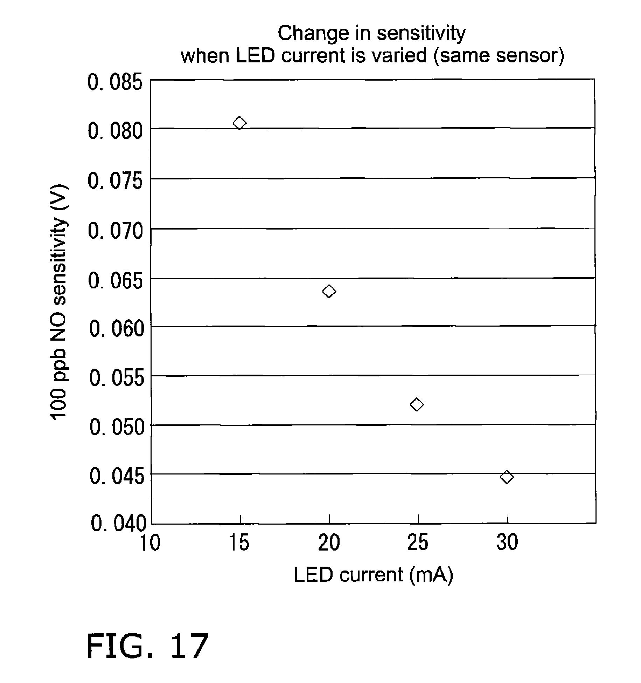

When a given NO concentration measurement sensor 230 is irradiated with light from the LED light source 211 in an environment with an NO concentration of 100 ppb, it can be seen from experimental results that the NO detection sensitivity changes along with an increase or decrease in the LED current applied to the LED light source 211 (the LED light quantity emitted from the LED light source 211). More specifically, as shown in FIG. 17, when the value of the LED current is increased to 25 and 30 mA, the NO detection sensitivity decreases to 0.052 and 0.044 V. Conversely, when the value of the LED current is decreased to 20 and 15 mA, the NO detection sensitivity rises to 0.064 and 0.081 V. The relation between NO detection sensitivity and LED current value has the correlation shown in FIG. 17. That is, it can be seen from the experimental results in FIG. 17 that by adjusting the LED current value, it is possible to control the amount of light emitted from the LED light source 211, and NO detection sensitivity can be controlled to some extent.

With the nitrogen oxide concentration measurement device 210 of this embodiment, in light of the above, the LED light quantity emitted from the LED light source 211 via the constant current drive circuit 212 is subjected to feedback control so that the 415 nm light quantity will remain constant during introduction of the initial NO concentration gas detected at the 415 nm light quantity detector 216.

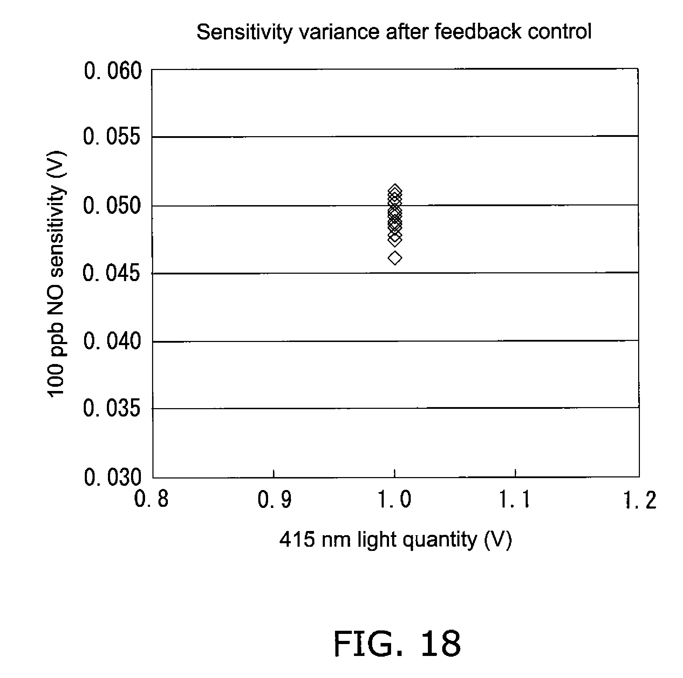

Under the same conditions as in FIG. 16 (in an environment with an NO concentration of 100 ppb), light with the same LED light quantity was emitted from the LED light source 211 at the same 15 NO concentration measurement sensors 230 as in FIG. 16, and feedback control was performed so that the 415 nm light quantity during introduction of gas with an NO concentration of 0 ppb would be a constant value (1.0 V in FIG. 18), and as a result, it can be seen from the experimental results that there is less variance in the NO detection sensitivity after feedback control obtained for the NO concentration measurement sensors 230. More specifically, the 415 nm light quantity is fixed at approximately 1.0 V, the NO detection sensitivity falls within a range of approximately 0.052 V to approximately 0.046 V, and there is little variance.

With the experimental results shown in FIG. 18, it can be seen that the CV value for sensitivity variance is 2.73%, which is greatly improved over the CV value shown in FIG. 16 (6.43%), and reproducibility is better.

In light of the above, the concentration of NO contained in exhaled air was actually measured using the above configuration, and the flow of control up to the correction of the measurement value will now be described in detail.

Working Example 1

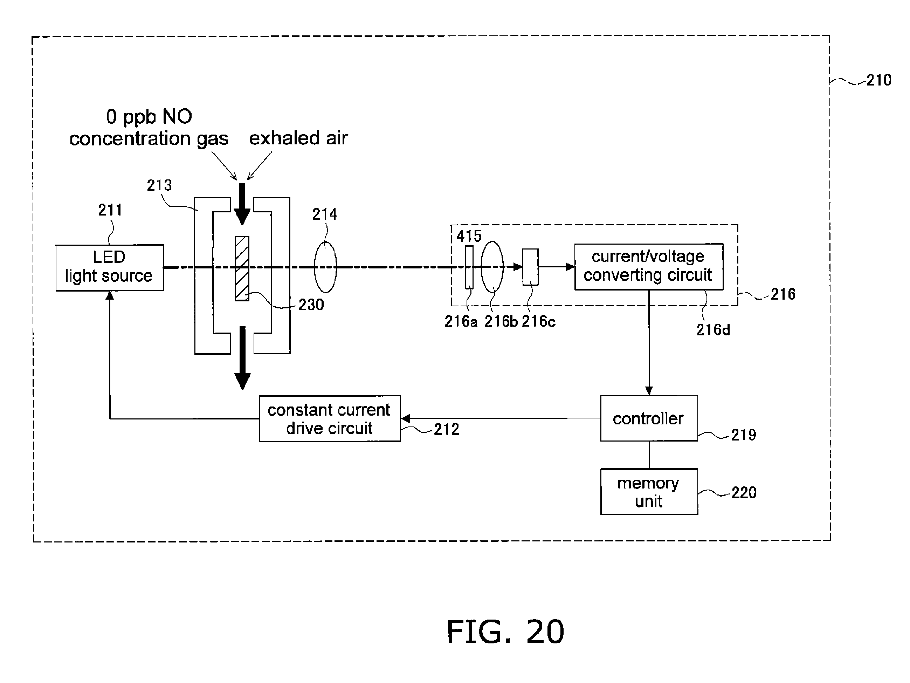

With the nitrogen oxide concentration measuring device 210 in this example, using the configuration shown in FIG. 20, first the 415 nm light quantity is detected with the 415 nm light quantity detector 216 out of the light transmitted through the portion of the NO concentration measurement sensor 230 where the porphyrin is supported, in a state in which air with an NO concentration of 0 ppb is introduced into the chamber 213.

Next, the 415 nm light quantity detector 216 outputs this detection result to the controller 219.

Next, the controller 219 performs feedback control over the LED light quantity emitted from the LED light source 211 so that the voltage value indicating the detected 415 nm light quantity will be a preset target value. More specifically, the current applied from the constant current drive circuit 212 to the LED light source 211 is controlled so that the 415 nm light quantity (voltage value) during introduction of gas with an NO concentration of 0 ppb will be a specific, preset target value (such as 1.0 V).

This feedback control may be such that a specific value serving as a target value (such as 1.0 V) is reached in a single feedback, or such that the feedback control is split up into a plurality of times and the target value is gradually approached.

Next, as a result of the feedback control, the controller 219 controls the constant current drive circuit 212 so that the corrected LED current for substantially matching the 415 nm light quantity to the target value is applied to the LED light source 211.

Next, in a state in which exhaled air has been introduced into the chamber 213, light corresponding to the corrected LED current is emitted from the LED light source 211, and the 415 nm light quantity is detected at the 415 nm light quantity detector 216.

Then, using a mathematical formula corresponding to a calibration curve pre-stored in the memory unit 220, the differential output at the 415 nm light quantity detector 216 during introduction of a gas with an NO concentration of 0 ppb and during the introduction of exhaled air is converted into an NO concentration value (ppb).

Since the NO concentration value thus obtained is a measurement result in a state in which variance in the NO detection sensitivity shown in FIG. 18 has been suppressed by the above-mentioned feedback control, this value can be acquired as a very accurate, corrected NO concentration.

As discussed above, with the nitrogen oxide concentration measuring device 210 in this embodiment, feedback control is performed on the LED light quantity on the basis of the 415 nm light quantity, which makes it possible to reduce variance in detection sensitivity for each NO concentration measurement sensor 230 that is caused by variance in the amount of porphyrin impregnation, etc., attributable to variance in the thickness of the base material 231 in the NO concentration measurement sensor 230, for example. That is, performing the above-mentioned correction control allows a specific measurement of the amount of porphyrin absorption in the NO concentration measurement sensor 230, etc., to be carried out equivalently.

Also, if variance in the emitted LED light quantity occurs even though the same LED current is applied, due to the temperature characteristics of the LED light source 211, aging, individual variance, or the like, feedback control is performed on the LED light quantity emitted from the LED light source 211 so that the 415 nm light quantity will be a constant target value, so the measurement of NO concentration can be carried out in a state in which such variance is also absorbed.

As a result, very accurate NO concentration measurement can be performed by correcting for measurement error caused by variance in the amount of porphyrin absorption from one NO concentration measurement sensor 230 to the next, the temperature characteristics of the LED light source 211, aging, and so forth.

Also, instead of using a 415 nm wavelength, the NO sensitivity may be calculated by detecting the amount of light with a wavelength of 435 nm, which reacts in opposite phase to that of the 415 nm wavelength.


Working Example 2

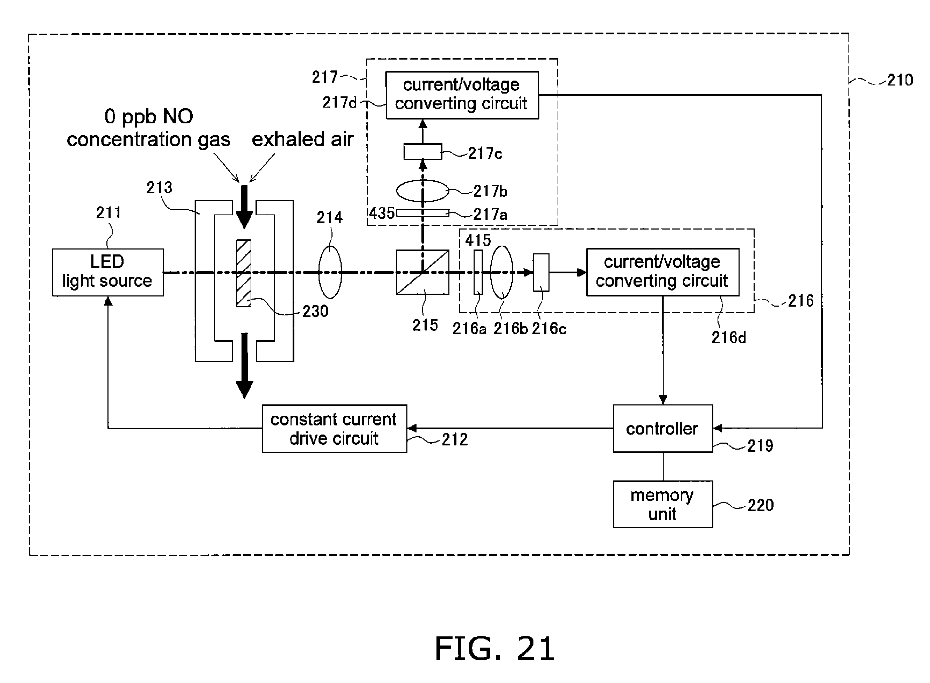

In this example, using the configuration shown in FIG. 21, first the 415 nm light quantity is detected with the 415 nm light quantity detector 216 out of the light transmitted through the portion of the NO concentration measurement sensor 230 where the porphyrin is supported, in a state in which air with an NO concentration of 0 ppb is introduced into the chamber 213.

Next, the 415 nm light quantity detector 216 outputs this detection result to the controller 219.

Next, the controller 219 performs feedback control over the LED light quantity emitted from the LED light source 211 so that the voltage value indicating the detected 415 nm light quantity will be a preset target value. More specifically, the current applied from the constant current drive circuit 212 to the LED light source 211 is controlled so that the 415 nm light quantity (voltage value) during introduction of gas with an NO concentration of 0 ppb will be a specific, preset target value (such as 1.0 V).

This feedback control may be such that a specific value serving as a target value (such as 1.0 V) is reached in a single feedback, or such that the feedback control is split up into a plurality of times and the target value is gradually approached.

Next, as a result of the feedback control, the controller 219 controls the constant current drive circuit 212 so that the corrected LED current for substantially matching the 415 nm light quantity to the target value is applied to the LED light source 211.

Next, in a state in which exhaled air has been introduced into the chamber 213, light corresponding to the corrected LED current is emitted from the LED light source 211, and the 435 nm light quantity is detected at the 435 nm light quantity detector 217.

The detected output is converted into an NO concentration value (ppb) using a mathematical formula corresponding to a calibration curve pre-stored in the memory unit 220.

In this example, as discussed above, the amount of light of a specific wavelength (415 nm) is detected, feedback control is performed, the LED light quantity emitted from the LED light source 211 is controlled so as always to be constant, and in this state the NO sensitivity is calculated on the basis of the output difference in the amount of light of another wavelength (435 nm) before and after the introduction of exhaled air (NO exposure).

Consequently, it is possible to suppress variance in detection sensitivity from one NO concentration measurement sensor 230 to the next caused by variance in the amount of porphyrin impregnation attributable to the variance in the thickness of the base material 31 in the NO concentration measurement sensor 230, etc. That is, performing the above-mentioned feedback control allows a specific measurement of the amount of porphyrin absorption in the NO concentration measurement sensor 230, etc., to be carried out equivalently.

Also, if variance in the emitted LED light quantity occurs even though the same LED current is applied, due to the temperature characteristics of the LED light source 211, aging, individual variance, or the like, feedback control is performed on the LED light quantity  emitted from the LED light source 211 so that the 415 nm light quantity will be a constant target value, so the measurement of NO concentration can be carried out in a state in which such variance is also absorbed.

As a result, very accurate NO concentration measurement can be performed by correcting for measurement error caused by variance in the amount of porphyrin absorption from one NO concentration measurement sensor 230 to the next, the temperature characteristics of the LED light source 211, aging, and so forth.

Also, the NO sensitivity may be calculated on the basis of the output difference in the amount of light with a wavelength of 415 nm before and after the introduction of exhaled air (NO exposure) in a state in which the amount of light when the NO concentration is 0 ppb and the wavelength is 435 nm, and the LED light quantity emitted from the LED light source 211 is always kept constant.

Working Example 3

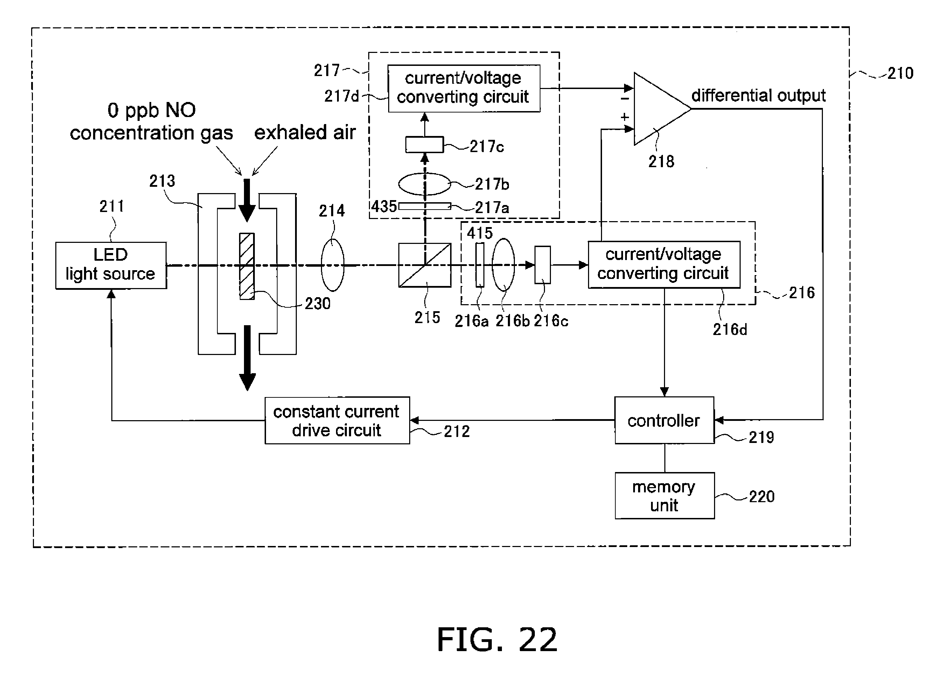

In this embodiment, using the configuration shown in FIG. 22, first the 415 nm light quantity is detected with the 415 nm light quantity detector 216 out of the light transmitted through the portion of the NO concentration measurement sensor 230 where the porphyrin is supported, in a state in which air with an NO concentration of 0 ppb is introduced into the chamber 213.

Next, the 415 nm light quantity detector 216 outputs this detection result to the controller 219.

Next, the controller 219 performs feedback control over the LED light quantity emitted from the LED light source 211 so that the voltage value indicating the detected 415  nm light quantity will be a preset target value. More specifically, the current applied from the constant current drive circuit 212 to the LED light source 211 is controlled so that the 415 nm light quantity (voltage value) during introduction of gas with an NO concentration of 0 ppb will be a specific, preset target value (such as 1.0 V).

This feedback control may be such that a specific value serving as a target value (such as 1.0 V) is reached in a single feedback, or such that the feedback control is split up into a plurality of times and the target value is gradually approached.

Next, as a result of the feedback control, the controller 219 controls the constant current drive circuit 212 so that the corrected LED current for substantially matching the 415 nm light quantity to the target value is applied to the LED light source 211.

Next, in a state in which exhaled air has been introduced into the chamber 213, light corresponding to the corrected LED current is emitted from the LED light source 211, and the 415 nm light quantity and the 435 nm light quantity are detected at the 415 nm light quantity detector 216 and the 435 nm light quantity detector 217.

The sum of the absolute values for the amount of change in the detected 415 nm light quantity and the 435 nm light quantity, or the differential output, is converted into an NO concentration value (ppb) using a mathematical formula corresponding to a calibration curve pre-stored in the memory unit 220.

In this example, as discussed above, the NO sensitivity is calculated on the basis of the output difference in the amount of light of two wavelengths (415 and 435 nm) before and after the introduction of exhaled air (NO exposure), in a state in which the amount of light of  a specific wavelength (415 nm) is detected, feedback control is performed, and the LED light quantity emitted from the LED light source 211 is always kept constant.

Consequently, it is possible to suppress variance in detection sensitivity from one NO concentration measurement sensor 230 to the next caused by variance in the amount of porphyrin impregnation attributable to the variance in the thickness of the base material 31 in the NO concentration measurement sensor 230, etc. That is, performing the above-mentioned feedback control allows a specific measurement of the amount of porphyrin absorption in the NO concentration measurement sensor 230, etc., to be carried out equivalently.

Also, if variance in the emitted LED light quantity occurs even though the same LED current is applied, due to the temperature characteristics of the LED light source 211, aging, individual variance, or the like, feedback control is performed on the LED light quantity emitted from the LED light source 211 so that the 415 nm light quantity will be a constant target value, so the measurement of NO concentration can be carried out in a state in which such variance is also absorbed. Furthermore, it is possible to reduce common mode noise of 435 nm and 415 nm in the measurement of NO concentration.

As a result, very accurate NO concentration measurement can be performed by correcting for measurement error caused by variance in the amount of porphyrin absorption from one NO concentration measurement sensor 230 to the next, the temperature characteristics of the LED light source 211, aging, and so forth.

Also, the NO sensitivity may be calculated on the basis of the output difference in the amount of light with a wavelength of 435 nm and the amount of light with a wavelength of 415 nm before and after the introduction of exhaled air, rather than the output difference in  the amount of light with a wavelength of 415 nm and the amount of light with a wavelength of 435 nm before and after the introduction of exhaled air.

Working Example 4

In this example, just as in Working Example 3 above, using the configuration shown in FIG. 22, the NO sensitivity is calculated on the basis of the sum of the absolute values of the output difference (differential output) in the amount of light with a wavelength of 415 nm and the amount of light with a wavelength of 435 nm before and after the introduction of exhaled air (NO exposure), while feedback control is performed on the LED light quantity of the LED light source 211, so that the amount of light with a wavelength of 415 nm outputted from the current/voltage conversion circuit 216d will always be constant.

Here again, the same effect as in the above examples can be obtained.

In the feedback control discussed above, the NO sensitivity may be calculated on the basis of the sum of the absolute values of the output difference (differential output) in the amount of light with a wavelength of 415 nm and the amount of light with a wavelength of 435 nm before and after the introduction of exhaled air (NO exposure), while feedback control is performed on the LED light quantity of the LED light source 211, so that the amount of light with a wavelength of 435 nm outputted from the current/voltage conversion circuit 217d of the 435 nm light quantity detector 217 will always be constant.

Other Embodiments

Embodiments of the present invention were described above, but the present invention is not limited to or by the above embodiments, and various modifications are possible without departing from the gist of the invention.

(A) In the above embodiment, an example was given in which the NO concentration was measured using an NO concentration measurement sensor supporting porphyrin containing cobalt as the central metal. However, the present invention is not limited to this.

For example, an NO concentration measurement sensor supporting porphyrin containing another metal as the central metal may be used instead.

Here again, the NO concentration can be measured by detecting the amount of change in the absorption spectrum of light with a specific wavelength after a reaction with NO.

(B) In the above embodiment, an example was given in which the NO concentration was measured by detecting transmitted light emitted at an NO concentration measurement sensor supporting porphyrin containing cobalt as the central metal, but the present invention is not limited to this.

For example, a sensor including a base material that reflects emitted light can also be used.

In this case, the base material of the sensor can be any one that reflects light, such as alumina, silicon, silicon carbide, gallium arsenide, ceramic, plastic, metal, or the like.

(C) In the above embodiment, an example was given of using an NO concentration measurement sensor supporting porphyrin on a base material 31 composed of paper or cellulose fibers, but the present invention is not limited to this.

For example, a detecting film that contains porphyrin but has no base material may be used as the NO concentration measurement sensor.

(D) In the above embodiment, an example was given in which the NO concentration was measured by using an NO concentration measurement sensor 230 containing porphyrin (CoT(dtBu)PP), but the present invention is not limited to this.

For example, even when using an NO concentration measurement sensor containing a metal complex of a derivative of porphyrin, the same effect as above can be obtained by measuring the amount of light with a peak wavelength in the absorption spectrum that varies as a result of reaction with NO.

(E) In the above embodiment, an example was given in which the concentration of NO (nitric oxide) contained in exhaled air was measured in the nitrogen oxide concentration measurement device of the present invention, but the present invention is not limited to this.

For example, with the nitrogen oxide concentration measurement device of the present invention, it is also possible to measure the concentration of nitrogen dioxide.

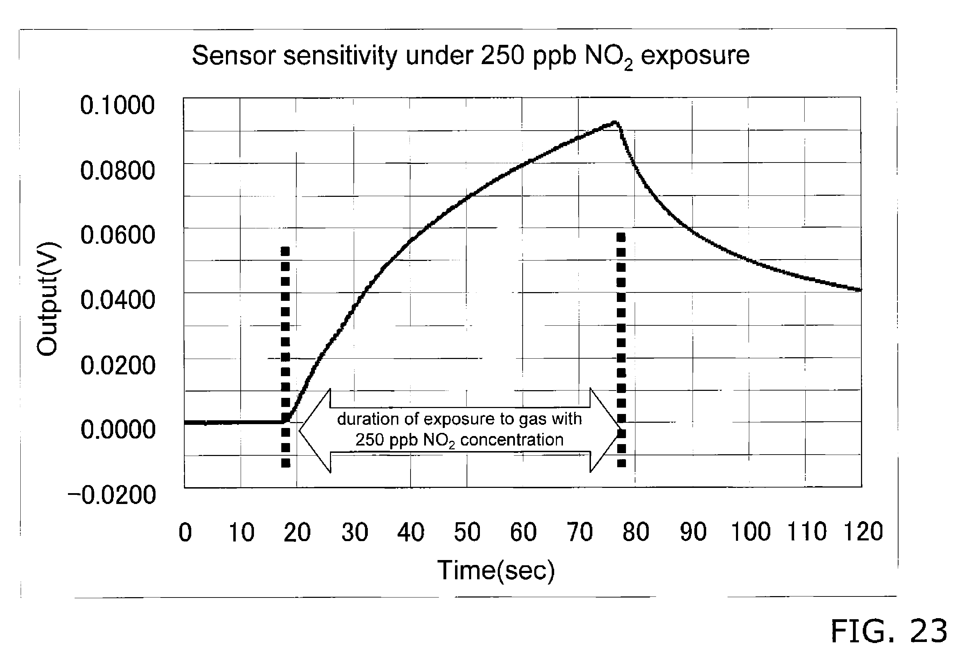

FIG. 23 is a graph of sensor sensitivity resulting from exposure to 250 ppb of $NO_2$.

As shown in FIG. 23, with the nitrogen oxide concentration measurement device of the present invention, a reaction with nitrogen dioxide can be detected in the same manner as with nitric oxide. Thus, with the nitrogen oxide concentration measurement device of the present invention, not just an NO concentration, but also an $NO_2$ concentration can be measured.

Working Example 5

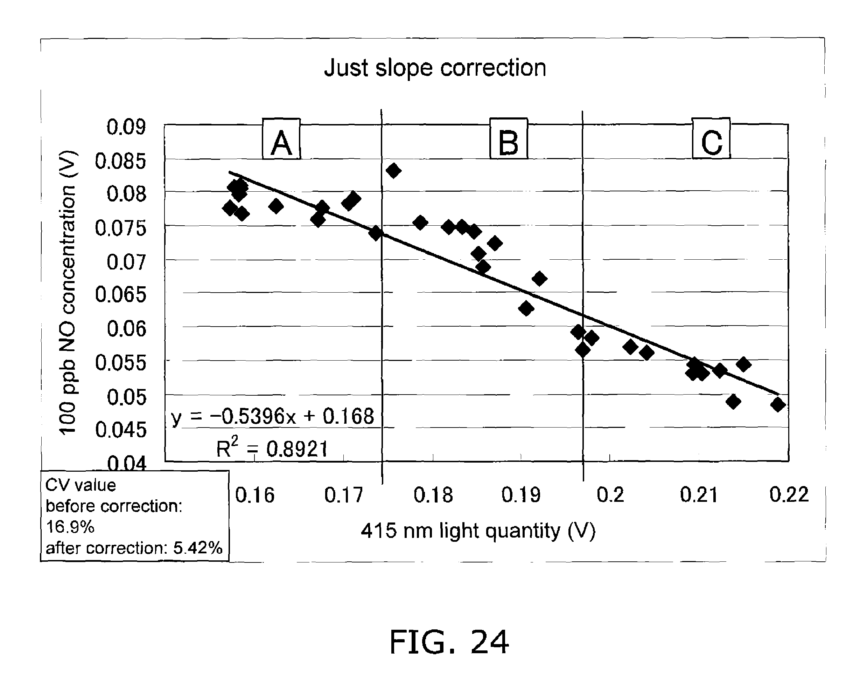

As shown in FIG. 24, in this example we will describe an example of correcting sensitivity variance, using a graph of the relation between 100 ppb NO sensitivity and the 415 nm light quantity detected by a 415 nm light quantity detector.

More specifically, in this example, the horizontal axis (415 nm light quantity) in the graph is divided into a plurality of zones and slope correction is performed in each of these zones, as an application example of the slope correction of a correlation line described in Embodiment 1 above.

Specifically, in this example 34 NO concentration measurement sensors were produced under the same conditions, and the 100 ppb NO sensitivity was measured for each, which gave the results shown in FIG. 24.

Here, the CV value indicating the sensitivity variance when slope correction was performed for all data was 5.42%.

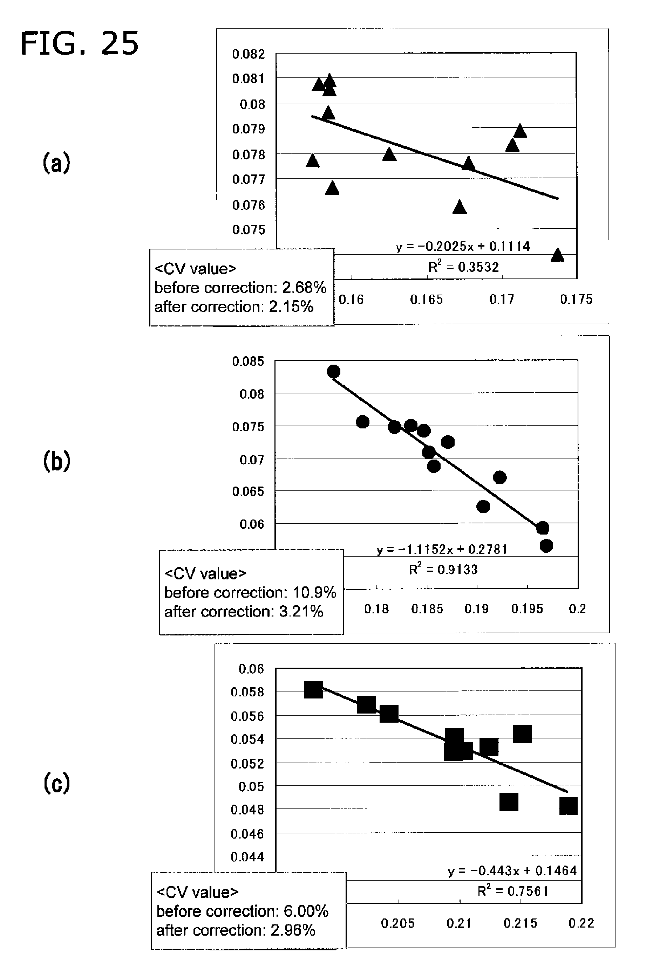

Next, the horizontal axis side of the graph in FIG. 24 at (415 nm light quantity) was divided into three zones (A zone, B zone, and C zone), and slope correction was performed in each zone as shown in FIGS. 25a to 25c.

Here, the graph in FIG. 25a corresponds to the A zone in the graph of FIG. 24, the graph in FIG. 25b to the B zone in the graph of FIG. 24, and the graph in FIG. 25c to the C zone in the graph of FIG. 24.

As a result, as shown in FIGS. 25a to 25c, the CV value of the sensitivity variance in each zone was 2.15% in the A zone, 3.12% in the B Zone, and 2.96% in the C Zone. That is, when slope correction was performed on the measurement results included in the graph divided into three zone along the horizontal axis, it was found that accuracy of sensitivity correction could be markedly improved to CV values of 2.15 to 3.12%, compared to the original (undivided) data for slope correction (CV value of 5.42%).

Specifically, it was found that when the horizontal axis side in the graph of FIG. 24 is divided into a plurality of zones at 415 nm light quantity and slope correction is performed in each zone, sensitivity variance can be suppressed by using individual calibration curves for each of the zones A to C.

Working Example 6

In this example, as an application example of the feedback correction described in Embodiment 3 above, we will describe an example of performing this feedback correction in each of the zones obtained by dividing the horizontal axis of the graph (415 nm light quantity) into a plurality of zones.

Specifically, in this example, 34 NO concentration measurement sensors were produced, the 100 ppb NO sensitivity was measured for each, and slope correction was performed, whereupon the CV value for sensitivity variance was 5.42%.

Next, in the same manner as in Working Example 5, the out of 415 nm during zero gas exposure (such as the horizontal axis shown in FIG. 24) was divided into three zones (A, B, and C), feedback control was applied to the measurement result in each zone so that the amount of light with a wavelength of 415 nm would be 0.16 V in the A zone, 0.185 V in the B zone, and 0.21 V in the C zone, and the 100 ppb NO sensitivity was measured again.

As a result, the CV values in the various zones was 2.19% in the A zone, 2.51% in the B zone, and 1.86% in the C zone. That is, it was found that accuracy of sensitivity correction could be markedly improved to CV values of 1.86 to 2.19%, compared to the original (undivided) data for slope correction (CV value of 5.42%).

For example, in the graph shown in FIG. 24, when the 415 nm light quantity during initial zero gas exposure was divided into a plurality of zones, and feedback control was performed in each of these divided zones, it was found that sensitivity variance could be reduced by using individual calibration curves for the measurement results in each of the zones.

INDUSTRIAL APPLICABILITY

The effect of the nitrogen oxide concentration measurement device of the present invention is that detection error caused by changes in the amount of light due to aging, the temperature characteristics of the light source, or variance in sensitivity from one sensor to the next can be suppressed, and nitrogen oxide concentrations can be measured more accurately, which means that the present invention can be widely applied to sensors that measure the concentration of nitrogen oxides.

REFERENCE SIGNS LIST

10        nitrogen oxide concentration measurement device

| | |
|---|---|
| 11 | LED light source (light source) |
| 12 | constant current drive circuit |
| 13 | chamber |
| 13a | gas inlet |
| 13b | gas outlet |
| 14 | lens |
| 15 | dichroic mirror |
| 16 | 415 nm light quantity detector (light detector, first detector) |
| 16a | bandpass filter |
| 16b | lens |
| 16c | photodiode |
| 17 | 435 nm light quantity detector (light detector, second detector) |
| 17a | bandpass filter |
| 17b | lens |
| 17c | photodiode |
| 17d | current/voltage converting circuit |
| 18 | differential processor |
| 19 | controller |
| 20 | memory unit |
| 30 | NO concentration measurement sensor |
| 31 | base material |
| 110 | nitrogen oxide concentration measurement device |
| 111 | mouthpiece |
| 112 | NO scrubber |
| 113 | zero scrubber |
| 114 | dryer |
| 115 | chamber |
| 115a | gas inlet |
| 115b | gas outlet |
| 116 | valve |
| 117 | valve |
| 118 | valve |
| 119 | compressor |
| 121 | LED light source (light source) |
| 122 | constant current drive circuit |
| 123 | lens |
| 124 | dichroic mirror |
| 125 | 415 nm transmitted light quantity detector |
| 125a | bandpass filter |
| 125b | lens |
| 125c | photodiode |
| 125d | current/voltage conversion circuit |
| 126 | 435 nm transmitted light quantity detector |
| 126a | bandpass filter |
| 126b | lens |
| 126c | photodiode |
| 126d | current/voltage conversion circuit |
| 127 | differential amplifier circuit |
| 128 | controller |
| 129 | memory unit |
| 130 | NO concentration measurement sensor |
| 181 | first path |
| 182 | second path |
| 183 | third path |
| 210 | nitrogen oxide concentration measuring device |
| 211 | LED light source (light source) |
| 212 | constant current drive circuit |
| 213 | chamber |

| 213a | gas inlet |
| 213b | gas outlet |
| 214 | lens |
| 215 | dichroic mirror |
| 216 | 415 nm light quantity detector (light detector, first detector) |
| 216a | bandpass filter |
| 216b | lens |
| 216c | photodiode |
| 217 | 435 nm light quantity detector (light detector, second detector) |
| 217a | bandpass filter |
| 217b | lens |
| 217c | photodiode |
| 217d | current/voltage converting circuit |
| 218 | differential processor |
| 219 | controller |
| 220 | memory unit |
| 230 | NO concentration measurement sensor |

**Claims**

1. A nitrogen oxide concentration measurement device measures a nitrogen oxide concentration by shining light on a sensor produced by distributing a metal complex of porphyrin or a derivative thereof over a base material, and detecting the amount of light of a specific wavelength, said device comprising:

   a light source that shines light at a nitrogen oxide concentration measurement sensor,;
   a light detector that detects the amount of light of a specific wavelength out of all the light transmitted or reflected by the nitrogen oxide concentration measurement sensor, and
   a controller that corrects the detection result at the light detector so as to match the amount of light of a specific wavelength outputted from the light detector during exposure to a zero gas to a corrected value.

2. The nitrogen oxide concentration measurement device according to Claim 1,
   further comprising a memory unit configured to pre-store correlation information indicating a relation between a slope of the correlation lines indicating the relation between an amount of light of a specific wavelength and the nitrogen oxide concentration, and a nitrogen oxide concentration detection sensivity;
   wherein the controller amends a value of the detection result detected in the light detector by using the slope of the correlation lines obtained on the basis of the correlation information stored in the memory unit so that a measurement value in the light detector during exposure to the zero gas is a predetermined target value when a light of the specific wavelength is detected in the light detector.

3. The nitrogen oxide concentration measurement device according to Claim 1, further comprising:

   a calibration information selector configured to select a calibration information used for concentration determination on the basis of the signal from the light detector during exposure to the zero gas from two or more types of calibration information indicating a relation between the signal and the nitrogen oxide concentration;
   wherein the controller determines the nitrogen oxide gas concentration in the measurement gas on the basis of the correlation information selected by the correlation information selector.

4. The nitrogen oxide concentration measurement device according to Claim 1,
   wherein the controller determines the predetermined target value on the basis of the correlation information selected by the correlation information selector.

5. The nitrogen oxide concentration measurement device according to Claim 1,
   which the controller feedback controls to adjust the amount of the light shined from the light source so that the amount of the light gets to be the predetermined target value , and measures the nitrogen oxide concentration on the basis of the detected value after feedback control detected in the light detector from the amount of the light after adjusting the amount of the light shined from the light source when the amount of light of a specific wavelength is detected in the light detector.

**6.** The nitrogen oxide concentration measurement device according to Claim 5,
wherein the controller determines the predetermined target value on the basis of the calibration information selected by the calibration information selector.

FIG. 1

FIG. 2

FIG. 3

drip nozzle
inside diameter 200 μm

solution (10 μL)

base material (paper approximately 200 μm thick) 31

# FIG. 4

Sensitivity variance during exposure to NO at 0, 100, and 200 ppb

FIG. 5

EP 2 685 241 A1

FIG. 6

FIG. 7

EP 2 685 241 A1

Height dependence of 0.1 ppm NO sensitivity

FIG. 8

EP 2 685 241 A1

EP 2 685 241 A1

# FIG. 9

Height dependence of correlation (R2 value)
between 415 nm light quantity and 0.1 ppm NO sensitivity

R2 value

Nozzle position (μm)

EP 2 685 241 A1

air →  compressor 119

zero scrubber 113

181

mouthpiece 111

116

117

dryer 114

chamber 115

182

183

NO scrubber 112

110

FIG. 10

FIG. 11

```
                    ┌─────────┐
                    │  Start  │
                    └─────────┘
                         │
                         ▼
S1 ─────────┌──────────────────────────┐
            │      Initialization       │
            └──────────────────────────┘
                         │
                         ▼
S2 ─────────┌──────────────────────────┐
            │  Introduction of zero gas │
            └──────────────────────────┘
                         │
                         ▼
S3 ─────────┌──────────────────────────┐
            │ Acquisition of initial signal │
            └──────────────────────────┘
                         │
                         ▼
S4 ─────────┌──────────────────────────┐
            │ Selection of calibration curve │
            └──────────────────────────┘
                         │
                         ▼
S5 ─────┌──────────────────────────────────────┐
        │ Acquisition of first difference D1 (= $V_i1 - V_{ii}1$) │
        └──────────────────────────────────────┘
                         │
                         ▼
S6 ─────────┌──────────────────────────┐
            │ Introduction of exhaled air │
            └──────────────────────────┘
                         │
                         ▼
S7 ─────┌──────────────────────────────────────┐
        │ Acquisition of second difference D2 (= $V_i2 - V_{ii}2$) │
        └──────────────────────────────────────┘
                         │
                         ▼
S8 ─────┌──────────────────────────────────────┐
        │ Acquisition of concentration signal C (= D2 – D1) │
        └──────────────────────────────────────┘
                         │
                         ▼
S9 ─────┌──────────────────────────────────────┐
        │ Acquisition of concentration based on │
        │ calibration curve and concentration signal C │
        └──────────────────────────────────────┘
                         │
                         ▼
                    ┌─────────┐
                    │   End   │
                    └─────────┘
```

# FIG. 12

FIG. 13

EP 2 685 241 A1

FIG. 14

EP 2 685 241 A1

FIG. 15

Sensitivity variance prior to feedback control

FIG. 16

EP 2 685 241 A1

Change in sensitivity
when LED current is varied (same sensor)

FIG. 17

FIG. 18

FIG. 19

FIG. 20

EP 2 685 241 A1

EP 2 685 241 A1

FIG. 21

FIG. 22

FIG. 23

FIG. 24

# FIG. 25

(a)

y = −0.2025x + 0.1114
$R^2$ = 0.3532

<CV value>
before correction: 2.68%
after correction: 2.15%

(b)

y = −1.1152x + 0.2781
$R^2$ = 0.9133

<CV value>
before correction: 10.9%
after correction: 3.21%

(c)

y = −0.443x + 0.1464
$R^2$ = 0.7561

<CV value>
before correction: 6.00%
after correction: 2.96%

## INTERNATIONAL SEARCH REPORT

International application No.

PCT/JP2012/001388

**A. CLASSIFICATION OF SUBJECT MATTER**
*G01N21/78*(2006.01)i

According to International Patent Classification (IPC) or to both national classification and IPC

**B. FIELDS SEARCHED**

Minimum documentation searched (classification system followed by classification symbols)
G01N21/00-G01N21/83, G01N31/00-G01N31/22

Documentation searched other than minimum documentation to the extent that such documents are included in the fields searched
| | | | |
|---|---|---|---|
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)
JSTPlus/JMEDPlus/JST7580(JDreamII)

**C. DOCUMENTS CONSIDERED TO BE RELEVANT**

| Category* | Citation of document, with indication, where appropriate, of the relevant passages | Relevant to claim No. |
|---|---|---|
| Y | WO 2010/061536 A1 (Panasonic Corp.), 03 June 2010 (03.06.2010), paragraphs [0047] to [0058], [0090] to [0094] | 1-6 |
| Y | JP 63-011633 Y2 (Mitsubishi Electric Corp.), 05 April 1988 (05.04.1988), column 2, line 18 to column 3, line 4 | 1-6 |
| A | JP 9-281036 A (Biophotonics Information Laboratories, Ltd.), 31 October 1997 (31.10.1997), paragraphs [0014], [0018] to [0024], [0032] | 1 |

☒ Further documents are listed in the continuation of Box C.  ☒ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 02 April, 2012 (02.04.12) | 17 April, 2012 (17.04.12) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.

PCT/JP2012/001388

| C (Continuation). | DOCUMENTS CONSIDERED TO BE RELEVANT | |
|---|---|---|
| Category* | Citation of document, with indication, where appropriate, of the relevant passages | Relevant to claim No. |
| A | Nobuo NAKANO et al., "An Automatic Measurement of Formaldehyde in Air by a Monitoring Tape Method", Journal of the Chemical Society of Japan, vol.1998, no.7, 10 July 1998 (10.07. 1998), pages 506 to 510, passage 3 | 3 |
| A | JP 2007-240239 A (Yokogawa Electric Corp.), 20 September 2007 (20.09.2007), paragraph [0050] | 5 |

Form PCT/ISA/210 (continuation of second sheet) (July 2009)

| INTERNATIONAL SEARCH REPORT<br>Information on patent family members | | International application No.<br>PCT/JP2012/001388 |
|---|---|---|

| WO 2010/061536 A1 | 2010.06.03 | CN 102292631 A | 2011.12.21 |
|---|---|---|---|
| | | EP 2360465 A1 | 2011.08.24 |
| | | US 2011/228276 A1 | 2011.09.22 |
| JP 63-011633 Y2 | 1988.04.05 | JP 57186846 U | 1982.11.27 |
| JP 9-281036 A | 1997.10.31 | (Family: none) | |
| JP 2007-240239 A | 2007.09.20 | (Family: none) | |

Form PCT/ISA/210 (patent family annex) (July 2009)

**REFERENCES CITED IN THE DESCRIPTION**

**Patent documents cited in the description**

- JP H1062350 B
- WO 2010061536 A
- JP H9264768 B